# EUROPÄISCHE PATENTSCHRIFT

(11) **EP 1 144 476 B1**
(45) Veröffentlichungstag und Bekanntmachung des Hinweises auf die Patenterteilung: **03.12.2003**
(21) Anmeldenummer: 99964568.2
(22) Anmeldetag: 14.12.1999
(51) Int. Cl.: C08G 18/67, C08G 18/78, C08G 18/81, C09D 175/16

(54) **DURCH ADDITION AN ISOCYANATGRUPPEN ALS AUCH DURCH STRAHLUNGSINDUZIERTE ADDITION AN AKTIVIERTE C-C-DOPPELBINDUNGEN HÄRTBARE BESCHICHTUNGSMITTEL**
COATING AGENTS WHICH CAN BE HARDENED BY THE ADDITION OF ISOCYANATE GROUPS AS WELL AS BY THE RADIATION-INDUCED ADDITION OF ACTIVATED C-C DOUBLE COVALENT BONDS
MATERIAUX DE REVETEMENT DURCISSABLES PAR ADDITION DE GROUPES ISOCYANATES ET PAR ADDITION, INDUITE PAR RAYONNEMENT, DE DOUBLES LIAISONS C-C ACTIVEES

(30) Priorität: 23.12.1998 DE 19860041
(43) Veröffentlichungstag der Anmeldung: 17.10.2001
(73) Patentinhaber: BASF AKTIENGESELLSCHAFT, 67056 Ludwigshafen (DE)
(72) Erfinder: BRUCHMANN, Bernd, D-67251 Freinsheim (DE); BECK, Erich, D-68526 Ladenburg (DE); RENZ, Hans, D-67149 Meckenheim (DE); KÖNIGER, Rainer, D-67061 Ludwigshafen (DE); SCHWALM, Reinhold, D-67157 Wachenheim (DE); LOKAI, Matthias, D-67677 Enkenbach-Alsenborn (DE); REICH, Wolfgang, D-67133 Maxdorf (DE)
(86) Internationale Anmeldenummer: EP9909905
(87) Internationale Veröffentlichungsnummer: WO00039183

(56) Entgegenhaltungen:
- EP-A- 0 549 116
- EP-A- 0 683 189
- US-A- 5 739 251
- US-A- 5 767 220
- DATABASE WPI Section Ch, Week 198650 Derwent Publications Ltd., London, GB; Class A25, AN 1986-328404 XP002133237 & JP 61 243815 A (NIPPON SYNTHETIC CHEM IND CO), 30. Oktober 1986 (1986-10-30)

## Beschreibung

Die Erfindung betrifft Verbindungen mit Isocyanatgruppen oder verkappten Isocyanatgruppen, Allophanatgruppen und radikalisch polymerisierbaren C-C-Doppelbindungen, wobei die C-C-Doppelbindungen durch eine direkt daran gebundene Carbonylgruppe oder ein O-Atom in Etherfunktion aktiviert sind (nachstehend kurz als "Aktivierte Doppelbindungen" bezeichnet), abgeleitet von Polyisocyanaten und Alkoholen A, die neben der Alkoholgruppe noch eine Aktivierte Doppelbindung, tragen (Verbindungen I).

Weiterhin betrifft die Erfindung strahlungshärtbare Zubereitungen und Beschichtungsmittel, die die Verbindungen I enthalten, Verfahren zur Beschichtung mit diesen Stoffen sowie mit diesen Verfahren hergestellte beschichtete Gegenstände.

Beschichtungsmittel auf Basis von isocyanatgruppenhaltigen Verbindungen sind z.B. in Form von 2-K-Lacken allgemein bekannt (vgl. Kunststoff Handbuch, Band 7, Polyurethan, 2. Auflage, 1983, Carl-Hanser-Verlag München Wien, Seiten 540 - 561). Von den industriellen Verarbeitern von Beschichtungsmittelsystemen wie der Lackindustrie wird erwartet, daß die Beschichtungsmittelsysteme ein vielfältiges Anforderungsprofil erfüllen. Dies betrifft sowohl die Verarbeitungs- als auch die Gebrauchseigenschaften.

Bei den Verarbeitungseigenschaften kommt es wesentlich darauf an, daß die Beschichtungsmittelsysteme möglichst wenig Lösungsmittel enthalten, gleichzeitig jedoch eine niedrige Viskosität aufweisen. Die geringe Viskosität ist erforderlich, damit die Lacke problemlos mit üblichen Verfahren, z.B. durch Aufsprühen, auf die zu beschichtende Oberfläche aufgetragen werden können. Der Lösungsmittelgehalt dieser Lacke bereitet Probleme, da bei der Verarbeitung der Lacke technisch aufwendige Maßnahmen ergriffen werden müssen, um zu gewährleisten, daß die Lösungsmittel, die beim Auftrag und Trocknen der Lacke freigesetzt werden, nicht in die Atmosphäre gelangen.

Weiterhin sollen die mit den Beschichtungsmitteln versehenen Gegenstände durch Bestrahlung mit UV-Strahlung härtbar sein. Insbesondere soll sich die Härte nach kurzer Bestrahlung mit relativ geringen Strahlendosen sprunghaft erhöhen, ohne daß eine längere Bestrahlung noch einen merklichen Anstieg der Härte bewirken würde. Bei den Systemen des Standes der Technik läßt sich diese Härtung nur mit sehr hohen Strahlendosen bewirken, d.h. die erforderlichen Verweilzeiten in den vorhandenen Bestrahlungsanlagen sind noch zu lang. Es werden deshalb Systeme benötigt, die Gruppen aufweisen, die unter Einwirkung von Strahlung in einer Polymerisationsreaktion mit möglichst geringen Strahlendosen, d.h. kurzen Belichtungszeiten, praktisch quantitativ abreagieren.

Weiterhin werden von den Verarbeitern in zunehmendem Maße sogenannte dual cure Systeme gefordert. Diese Systeme zeichnen sich dadurch aus, daß sie sowohl strahlungshärtbar als auch durch einen zweiten, unabhängigen Härtungsmechanismus aushärtbar sind. Besonders gewünscht werden solche Systeme, die sich nach dem Auftrag des Beschichtungsmittel durch möglichst kurze Bestrahlung mit UV-Licht zu einem Film, der staubtrocken ist, vorhärten lassen. Dieser Film soll dann im Verlauf einiger Tage durch einfache Lagerung an der Luft bei Raumtemperatur oder unter Erwärmen weiter aushärten, bis ein harter Film entstanden ist, der die endgültigen gewünschten Gebrauchseigenschaften aufweist. Diese Art der zweistufigen Härtung ist deshalb von besonderer Bedeutung, weil es den Verarbeitern der Beschichtungsmittelsysteme die Möglichkeit gibt, in einem ersten Arbeitsschritt einen Gegenstand mit einem Film zu beschichten und diesen Film in einem zweiten Arbeitsschritt weiterzuverarbeiten, insbesondere dem bereits beschichteten Gegenstand nach der Bestrahlung unter Anwendung von Druck ein bestimmtes Profil zu verleihen. Die Filme bzw. Folien müssen also bei ihrer verformung im zweiten Arbeitsschritt bereits ausgehärtet sein, so daß sie bei der Verformung nicht an den Werkzeugen kleben bleiben, andererseits dürfen sie jedoch noch nicht so hart sein, daß sie bei der Dehnung und verformung reißen. Die so hergestellten beschichteten Gegenstände müssen danach noch eine Weile gelagert werden, bis die Beschichtung ihre endgültigen Gebrauchseigenschaften erreicht hat.

Was die Gebrauchseigenschaften angeht, so werden hier insbesondere folgende Anforderungen gestellt.
- Unempfindlichkeit gegenüber mechanischer Beanspruchung wie Zug, Dehnung, Schlägen, Kratzen oder Abrieb,
- Resistenz gegenüber Feuchtigkeit (z.B. in Form von Wasserdampf), Lösemitteln, Benzin und verdünnten Chemikalien, sowie chemischen Umwelteinflüssen wie schefelsaurer Regen, Pankreatin, Baumharz
- Beständigkeit gegenüber Umwelteinflüssen wie Temperaturschwankungen und UV-Strahlung,
- hoher Glanz der beschichteten Oberflächen,
- gute Haftung auf verschiedenartigen Untergründen wie mit Grundierungen, Füllern, Farbeffektschichten oder sonstigen Beschichtungen vorbeschichtete Substrate, sowie direkt auf Kunststoffen, Holz, Holzwerkstoffen, Papier, Glas, Keramik, Textilien, Leder oder Metall.
- Weiterhin gefordert sind die vollständige Aushärtbarkeit unbelichteter oder nicht strahlungshärtbarer Lackstellen z.B. in beschatteten Bereichen z.B. von dreidimensionalen Substraten wie Fahrzeugkarosserien oder Poren in Holz, Papier, Schäumen, Keramikmaterialien, in Lacken mit Strahlung absorbierenden Inhaltsstoffen wie Pigmente, UV-Absorber, Füllstoffe,und von Spritznebelniederschlägen. Die Aushärtung soll beim Lagern an Luft oder unter zusätzlichem Erwärmen bzw. Einbrennen erfolgen.

Die nicht vorveröffentlichten Anmeldungen DE-A-19741781 und DE-A-19814874 betreffen strahlungshärtbare, Urethangruppen enthaltende Präpolymere, die als Beschichtungsmittel Verwendung finden. Sie enthalten jedoch keine freien Isocyanatgruppen.

Aus US 5300615 und US 5128432 sind ebenfalls Polyurethane mit radikalisch polymerisierbaren Doppelbindungen bekannt, die jedoch ebenfalls keine freien Isocyanatgruppen tragen.

Die EP-A-549116 und DE-A-3819627 betreffen Verbindungen, die sowohl Isocyanatgruppen als auch radikalisch polymerisierbare C-C-Doppelbindungen enthalten. Diese Verbindungen werden hergestellt, indem man handelsübliche aliphatische Isocyanate zu solchen mit Uretdion-, Isocyanurat- oder Biuretgruppen enthaltenden dimerisiert bzw. trimerisiert und anschließend mit Hydroxyalkylacrylaten umsetzt. Nachteilig an diesen Systemen ist, daß sie eine sehr hohe Viskosität aufweisen und die Verarbeitung nur unter Zusatz hoher Lösemittelmengen erfolgen kann.

Aus der US 5739251 sind ebenfalls Urethane aus Alkoholen, enthaltend beta,gamma-ethylenisch ungesättigte Ethergruppen, die praktisch frei von Isocyanatgruppen sind, sowie von diesen Urethanen abgeleitete Allophanate bekannt.

Die vorgenannten beta, gamma-ungesättigten Verbindungen weisen jedoch insbesondere den Nachteil auf, daß sie eine hohe Viskosität aufweisen und für sich nicht UV-strahlungshärtbar sind.

JP-A 61 243 815 beschreibt thixotrope Harze, die urethangruppenhaltige Produkte auf Basis TDI, Allophanatgruppen und radikalisch polymerisierbare C=C-Doppelbindungen enthalten.

Nachteilig an derartigen Verbindungen ist, dass aromatische Systeme, wie beispielsweise TDI, unter Tageslicht- oder UV-Einfluß zur Vergilbung neigen und somit nicht zur Herstellung von Klarlacken und UV-härtbaren Lacken verwendbar sind.

Der vorliegenden Erfindung liegt deshalb die Aufgabe zugrunde, Verbindungen bereitzustellen, mit denen sich Beschichtungsmittelsysteme herstellen lassen, die das vorgenannte Anforderungsprofil im Hinblick auf die Verarbeitungs- und Gebrauchseigenschaften aufweisen. Insbesondere sollen die Beschichtungsmittelsysteme eine niedrige Viskosität bei geringem Lösungsmittelgehalt aufweisen und als dual cure Systeme einsetzbar sein, bei dem die Strahlungshärtung mit geringeren Strahlendosen vollständig (oder bis zur Klebfreiheit oder Kratzbeständigkeit der Filme) durchgeführt werden kann.

Die erfindungsgemäßen Verbindungen I sind im allgemeinen im wesentlichen frei von Uretdion-, Biuret- oder Isocyanuratgruppen.

Bevorzugte Verbindungen I sind somit solche der allgemeinen Formel I

OCN-R¹-(-R²-C(O)-R²-R¹-)ₙ-NCO I

in der
- n: eine ganze Zahl von 1 bis 10, bevorzugt 1 bis 5
- R¹: eine divalente aliphatische oder alicyclische C₂- bis C₂₀-Kohlenwasserstoffeinheit oder eine aromatische C₅- bis C₂₀ Kohlenwasserstoffeinheit
- R²: in jeder Wiederholungseinheit einmal für -NH- steht und einmal für N-C(O)-R³ , wobei R³ für einen von einem Alkohol durch Abstraktion des H-Atoms von der alkoholischen Hydroxylgruppe abgeleiteten Rest steht, wobei der Alkohol als funktionelle Gruppen neben der Alkoholgruppe noch eine Aktivierte Doppelbindung trägt, bedeutet.

Bei den Resten R¹ handelt es sich bevorzugt um solche, die sich durch Abstraktion der Isocyanatgruppe von üblichen aliphatischen oder aromatischen Polyisocyanaten ableiten. Bei den Diisocyanaten handelt es sich bevorzugt um aliphatische Isocyanate mit 4 bis 20 C-Atomen. Beispiele für übliche Diisocyanate sind aliphatische Diisocyanate wie Tetramethylendiisocyanat, Hexamethylendiisocyanat(1,6-Diisocyanatohexan), Octamethylendiisocyanat, Decamethylendiisocyanat, Dodecamethylendiisocyanat, Tetradecamethylendiisocyanat, Derivate des Lysindiisocyanates, Tetramethylxylylendiisocyanat, Trimethylhexandiisocyanat oder Tetramethylhexandiisocyanat, cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5- trimethyl-5-(isocyanatomethyl)cyclohexan(Isophorondiisocyanat), 1,3- oder 1,4-Bis(isocyanatomethyl)cyclohexan oder 2,4-, oder 2,6-Diisocyanato-1-methylcyclohexan sowie aromatische Diisocyanate wie 2,4- oder 2,6-Toluylendiisocyanat, m- oder p-Xylylendiisocyanat, 2,4'- oder 4,4'-Diisocyanatodiphenylmethan, 1,3- oder 1,4-Phenylendiisocyanat, 1-Chlor-2,4-phenylendiisocyanat, 1,5-Naphthylendiisocyanat, Diphenylen-4,4'-diisocyanat, 4,4'-Diisocyanato-3,3'-dimethyldiphenyl, 3-Methyldiphenylmethan-4,4'-diisocyanat, oder Diphenylether-4,4'-diisocyanat. Es können auch Gemische der genannten Diisocyanate vorliegen. Bevorzugt sind Hexamethylendiisocyanat, 1,3-Bis(isocyanatomethyl)cyclohexan, Isophorondiisocyanat Tetramethylxylylendiisocyanat und Di(isocyanatocyclohexyl)methan.

Bei den Alkoholen A, von denen sich der Rest R³ ableitet, handelt es sich z.B. um Ester aus α, β-ungesättigten Carbonsäuren, wie Acrylsäure, Methacrylsäure (im folgenden kurz "(Meth)Acrylsäure"), Crotonsäure, Acrylamidoglykolsäure, Methacrylamidoglykolsäure oder Vinylessigsäure und Polyolen mit vorzugsweise 2 bis 20 C-Atomen und wenigstens 2 Hydroxylgruppen, wie Ethylenglykol, Diethylenglykol, Triethylenglykol, 1,2-Propylenglykol, 1,3-Propylenglykol, Dipropylenglykol, Tripropylenglykol, 1,4-Butandiol, 1,5-Pentandiol, Neopentylglykol, 1,6-Hexandiol, 2-Methyl-1,5-pentandiol, 2-Ethyl-1,4-butandiol, 1,4-Dimethylolcyclohexan, Glycerin, Trimethylolethan, Trimethylolpropan, Trimethylolbutan, Pentaerythrit, Ditrimethylolpropan, Erythrit und Sorbit, sofern der Ester wenigstens eine, gegenüber Isocyanat reaktive OH-Gruppe aufweist. Weiterhin können sich die Reste R³ auch von den Amiden der (Meth-)Acrylsäure mit Aminoalkoholen z. B. 2-Aminoethanol, 3-Amino-1-propanol, 1-Amino-2-propanol oder 2-(2-Aminoethoxy)ethanol und den Vinylethern der vorgenannten Polyole ableiten, sofern sie noch eine freie OH-Gruppe aufweisen.

Weiterhin sind auch ungesättigte Polyether- oder Polyesterole oder Polyacrylat-polyole mit einer mittleren OH-Funktionalität von 2 bis 10 als Reaktivkomponenten geeignet.

Bevorzugt leiten sich die Reste R³ von Alkoholen wie 2-Hydroxyethyl(meth)acrylat, Hydroxypropyl(meth)acrylat, 1,4-Butandiolmono(meth)acrylat, Neopentylglykolmono(meth)acrylat, Glycerinmono- und di(meth)acrylat, Trimethylolpropanmono- und di(meth)acrylat, Pentaerythritdi- und -tri(meth)acrylat ab. Besonders bevorzugt ist der Alkohol A ausgewählt aus 2-Hydroxyethylacrylat, 2-Hydroxyethylmethacrylat, Hydroxypropylacrylat, 1,4-Butandiolmonoacrylat und 3-(Acryloyloxy)-2-hydroxypropylmethacrylat. Beispiele für Amide ethylenisch ungesättigter Carbonsäuren mit Aminoalkoholen sind Hydroxyalkyl(meth)acrylamide wie N-Hydroxymethylacrylamid, N-Hydroxymethylmethacrylamid, N-Hydroxyethylacrylamid, N-Hydroyxethylmethacrylamid, 5-Hydroxy-3-oxopentyl8meth)acrylamid, N-Hydroxyalkylcrotonamide wie N-Hydroxymethylcrotonamid oder N-Hydroxyalkylmaleinimide wie N-Hydroxyethylmaleinimid.

Die Isocyanatgruppen der Verbindungen 1 können auch in verkappter Form vorliegen. Als Verkappungsmittel für NCO-Gruppen eignen sich z.B. Oxime, Phenole, Imidazole, Pyrazole, Pyrazolinone, Diketopiperazine, Caprolactam, Malonsäureester oder Verbindungen, wie sie genannt sind in den Veröffentlichungen von Z.W. Wicks, Prog. Org. Coat. 3 (1975) 73 - 99 und Prog. Org. Coat 9 (1981), 3 - 28 sowie in Houben-Weyl, Methoden der Organischen Chemie, Bd. XIV/2, 61 ff. Georg Thieme Verlag, Stuttgart 1963.

Die Verbindungen I kommen bevorzugt in Form von Mischungen (Mischungen I) zur Anwendung, enthaltend
a1) 1 bis 100 Gew.-% Verbindungen I
a2) 0 bis 99 Gew.-% einer sonstigen Verbindung, die neben einer oder mehreren Isocyanatgruppen noch eine Gruppe, ausgewählt aus der Gruppe der Urethan-, Harnstoff-, Biuret-, Allophanat-, Carbodiimid-, Uretonimin-, Uretdion- und Isocyanuratgruppen enthält.
   Isocyanate, die neben den Verbindungen I in den Mischungen I enthalten sein können, sind aliphatische und aromatische Diisocyanate und insbesondere höherfunktionelle Polyisocyanate (Polyisocyanate a2) der folgenden Gruppen:
   a2.1) Isocyanuratgruppen aufweisende Polyisocyanate von aliphatischen, cycloaliphatischen, aromatischen und/oder araliphatischen Diisocyanaten, die auch zum Aufbau der Verbindungen I eingesetzt werden können. Die Isocyanato-Isocyanurate haben im allgemeinen einen NCO-Gehalt von 10 bis 30 Gew.-%, insbesondere 15 bis 25 Gew.-%, und eine mittlere NCO-Funktionalität von 2,6 bis 4,5. (Isocyanate a2.1)
      Vor allem kommen Isocyanurate der allgemeinen Formel (II) 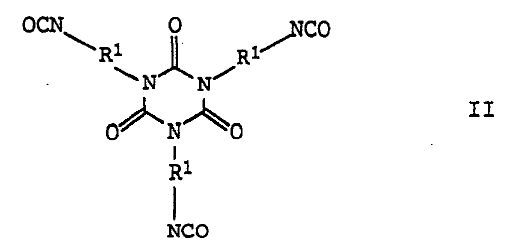 oder die sich davon ableitenden oligomeren Formen in Betracht, bei denen R¹ die gleiche Bedeutung wie bei Verbindungen der Formel I hat.
   a2.2) Uretdiongruppen enthaltende Diisocyanate mit aromatisch, aliphatisch und/oder cycloaliphatisch gebundenen Isocyanatgruppen, vorzugsweise von Hexamethylendiisocyanat oder Isophorondiisocyanat abgeleitet. Bei Poly-Uretdiondiisocyanaten handelt es sich um Dimerisierungsprodukte der -Diisocyanate (Isocyanate a2.2).
   a2.3) Biuretgruppen aufweisende Polyisocyanate mit aliphatisch gebundenen Isocyanatgruppen, insbesondere Tris(6-isocyanatohexyl)biuret oder dessen Gemische mit seinen höheren Homologen. Diese Biuretgruppen aufweisenden Polyisocyanate weisen im allgemeinen einen NCO-Gehalt von 10 bis 30 Gew.-%, insbesondere von 18 bis 25 Gew.-% und eine mittlere NCO-Funktionalität von 2,8 bis 4,5 auf (Isocyanate a2.3).
   a2.4) Urethan- und/oder Allophanatgruppen aufweisende Polyisocyanate, mit aliphatisch oder cycloaliphatisch gebundenen Isocyanatgruppen, die frei sind von radikalisch polymerisierbaren C-C-Doppelbindungen, wobei die C-C-Doppelbindungen durch eine direkt daran gebundene Carbonylgruppe oder ein O-Atom in Etherfunktion aktiviert sind. Derartige Verbindungen sind beispielsweise erhältlich durch Umsetzung von überschüssigen Mengen an Hexamethylendiisocyanat oder an Isophorondiisocyanat mit ein- oder mehrwertigen C₁- bis C₂₀-Monoalkoholen, mehrwertigen Alkoholen wie Ethylenglykol, Trimethylolpropan, Glycerin oder deren Gemischen. Diese Urethan- und/oder Allophanatgruppen aufweisenden Polyisocyanate haben im allgemeinen einen NCO-Gehalt von 12 bis 25 Gew.-% und eine mittlere NCO-Funktionalität von 2,5 bis 4,5 (Isocyanate a2.4)
   a2.5) Isocyanate, abgeleitet von einem Molekül eines Alkohols A und einem Molekül eines Polyisocyanats, wie es zur Herstellung der Verbindungen I eingesetzt wird (Isocyanate a2.5).
   a2.6) Oxadiazintriongruppen enthaltende Polyisocyanate, vorzugsweise von Hexamethylendiisocyanat oder Isophorondiisocyanat abgeleitet. Solche Oxadiazintriongruppen enthaltenden Polyisocyanate sind aus Diisocyanat und Kohlendioxid herstellbar. (Isocyanate a2.6)
   a2.7) Carbodiimid- oder Uretonimin-modifizierte Polyisocyanate (Isocyanate a2.7).

Die Isocyanatgruppen der genannten Polyisocyanate (a2.1) bis (a2.7) können auch teilweise mit Monoalkoholen umgesetzt sein.

Der Gehalt an Aktivierten Doppelbindungen der Verbindungen I, bezogen auf die Gesamtmenge der Mischungskomponenten a1 und a2 beträgt im allgemeinen 0,002 bis 20 Gew.-%,und bevorzugt 0,01 bis 10 Gew.-%.

Der Gehalt an Isocyanatgruppen der Komponenten a1 und a2, bezogen auf die Gesamtmenge der Mischungskomponenten a1 und a2. beträgt im allgemeinen 0.1 bis 40, und bevorzugt 1 - 30 Gew.%.

Bei der Angabe des Gehalts an Doppelbindungen wird eine Molmasse von 24 g/mol und bei der Angabe des Isocyanatgehalts in Gew.-% eine von 42 g/mol zugrunde gelegt.

Das Verhältnis der Aktivierten Doppelbindungen der Verbindungen I zu den Isocyanatgruppen der Mischungskomponenten a1 und a2 beträgt im allgemeinen 50 : 1 bis 0,02 : 1 und bevorzugt 10 : 1 bis 0,1 : 1.

Besonders bevorzugt sind solche Mischungen I, die die Komponenten a1 und a2.1 bis a2.7 in folgender Zusammensetzung enthalten:
Komponente a1: 5 - 95 Gew.%
Komponente a2.1: 5 - 60 Gew.%
Komponente a2.5: 0 - 60 Gew.%

Die Mischungen I weisen üblicherweise Viskositäten (gemessen bei 23°C) von weniger als 50000 mPas, bevorzugt von 100 - 30000 mPas auf.

Die Verbindungen I können hergestellt werden, indem man die Polyisocyanate und die Alkohole A, von denen sich die Verbindungen I ableiten, bei Reaktionstemperaturen von 0 bis 280, bevorzugt bei 20 bis 250°C, in Gegenwart eines die Allophanatbildung fördernden Katalysators, also beispielsweise einer Zink-organischen Verbindung, wie Zink-Acetylacetonat oder Zink-2-ethylcaproat, oder einer Tetraalkylammonium-Verbindung, wie N,N,N-Trimethyl-N-2-hydroxypropylammonium-hydroxid oder wie N,N,N-Trimethyl-N-2-hydroxypropylammonium-2-ethylhexanoat, umsetzt.

Die Mengen der Ausgangsverbindungen werden so gewählt, daß die Isocyanatgruppen im Überschuß vorliegen. Das Molverhältnis des eingesetzten Polyisocyanats zu eingesetztem Alkohol A beträgt im allgemeinen 1:1 bis 30 : 1, bevorzugt 1,5:1 bis 20:1.

Zur Stabilisierung der radikalisch polymerisierbaren Verbindungen (Verbindungen I) werden vorzugsweise 0,001 bis 2 Gew.-%, insbesondere 0,005 bis 1,0 Gew.-% Polymerisationsinhibitoren der Reaktion zugesetzt. Dabei handelt es sich um die üblichen, zur Behinderung der radikalischen Polymerisation geeigneten Verbindungen, z. B. um Hydrochinone oder Hydrochinonmonoalkylether, 2,6-Di-tert.-butylphenole, wie 2,6-Di-tert.-butylkresol, Nitrosamine, Phenothiazine oder Phosphorigsäureester.

Die Umsetzung kann sowohl lösungsmittelfrei als auch unter Zusatz von Lösungsmitteln durchgeführt werden. Als Lösungsmittel kommen inerte Lösungsmittel, z. B. Aceton, Methylethylketon, Tetrahydrofuran, Dichlormethan, Toluol, C₁-C₄-Alkylester der Essigsäure wie Ethylacetat oder Butylacetat in Betracht. Bevorzugt wird die Umsetzung lösungsmittelfrei durchgeführt.

Zunächst bilden sich bei der Umsetzung die entsprechenden Isocyanate a2.5 und hieraus die Verbindungen I mit einer Allophanatgruppe. Diese können zu Verbindungen mit mehr als einer Allophanatgruppe weiterragieren, beispielsweise den Verbindungen der Formel I, in denen n größer 1 ist.

Der Reaktionsfortschritt wird zweckmäßigerweise per Gelpermeationschromatographie (GPC) oder durch Bestimmung des NCO-Gehaltes der Reaktionsmischung verfolgt. Wenn die Reaktionsmischung die gewünschte Zusammensetzung erreicht hat, kann die Reaktion durch Zugabe von Desaktivatoren abgebrochen werden. Als Desaktivatoren eignen sich beispielsweise anorganische oder organische Säuren, die entsprechenden Säurehälogenide und Alkylierungsmittel. Beispielhaft genannt seien Phosphorsäure, Monochloressigsäure, Dodecylbenzolsulfonsäure, Benzoylchlorid, Dimethylsulfat und vorzugsweise Dibutylphosphat sowie Di-2-ethylhexylphosphat. Die Desaktivierungsmittel können in Mengen von 1 bis 200 Mol-%, vorzugsweise 20 bis 150 Mol-%, bezogen auf die Mole an Katalysator, eingesetzt werden. Meistens werden nach Beendigung der Umsetzung noch vorhandene Reste an unumgesetzten Polyisocyanat bis auf einen Gehalt von unter 0,5% im Vakuum abdestilliert.

Üblicherweise wird die Reaktion abgebrochen, wenn die Reaktionsmischung, sofern man etwaige noch vorhandene Mengen an eingesetztem Polyisocyanat außer acht läßt, folgende Zusammensetzung aufweist:
- Verbindung I mit 1 Allophanatgruppe: 1 bis 100, bevorzugt 5 bis 80 Gew.-%,
- Isocyanate a2.5: 0 bis 50, bevorzugt 0 bis 20 Gew.-%,
- Isocyanate a2.1: 0 bis 90, bevorzugt 0 bis 70 Gew.-%.

Aus den Reaktionsmischungen können die Verbindungen I mit Hilfe üblicher Trennmethoden isoliert werden, z.B. mit Hilfe der Gelpermeationschromatographie. Meistens ist dies jedoch nicht erforderlich, denn die Nebenprodukte sind ebenfalls Wertprodukte, die in Beschichtungsmittelsystemen, die die Verbindungen I enthalten, üblicherweise enthalten sein können.

Die Verbindungen I oder Mischungen I können zum einen durch Bestrahlung mit energiereicher Strahlung ggf. in Gegenwart anderer radikalisch polymerisierbarer Verbindungen mit C-C-Doppelbindungen ausgehärtet werden, wobei die C-C-Doppelbindungen der Verbindungen I und ggf. der sonstigen vorhandenen radikalisch polymerisierbaren Verbindungen polymerisiert werden.

Weiterhin können die Verbindungen I oder Mischungen I durch Reaktion der Isocyanatgruppen in einer Polyadditionsreaktion ausgehärtet werden (nachfolgend ''Isocyanathärtung'' genannt), z.B. indem man den Verbindungen I oder Mischungen I vor ihrer Anwendung weitere zusetzt, die mindestens eine gegenüber Isocyanat reaktiv Gruppe enthalten, die mit den Isocyanatgruppen in einer Additionsreaktion reagiert oder indem man in Beschichtungen aus den Verbindungen I oder Mischungen I derartige Verbindungen aus einem gasförmigen Medium eindiffundieren läßt.

Die Isocyanathärtung kann durch Erhöhung der Temperatur beschleunigt werden. Im allgemeinen sind hierfür Temperaturen bis 130°C geeignet, da es bei diesen Temperaturen möglich ist, nur die Isocyanathärtung zu bewirken, ohne daß die Polymerisation der C-C-Doppelbindung einsetzt.

Für den Fall, daß die Isocyanatgruppen in verkappter Form vorliegen, ist es meist ebenfalls erforderlich, die Isocyanatreaktion bei Temperaturen von 40 bis 200°C durchzuführen, um die Schutzgruppen abzuspalten.

Die Verbindungen oder Mischungen I können bereits ohne weitere Zusätze als Beschichtungsmittel und speziell als dual cure Systeme eingesetzt werden, denn Filme dieser Beschichtungsmittel können sowohl mit Hilfe von energiereicher Strahlung, gegebenenfalls unter Zusatz von Photoinitiatoren, als auch mittels Isocyanathärtung ausgehärtet werden. Die Isocyanathärtung der Beschichtungen kann auch z.B. in Kontakt mit einem Medium, das eine gegenüber Isocyanatgruppen reaktive Substanz (W) enthält, erfolgen. Beispielsweise kann Substanz (W) in Form eines Gases aus der Umgebungsatmosphäre, oder einer Flüssigkeit oder einer Substanz, die beispielsweise auf einem festen Trägermaterial aufgebracht ist auf Filme der Beschichtungsmittel einwirken. Beispiele wären Wasserdampf, Ammoniak oder Amine, die aus der Gasphase absorbiert werden und reagieren. Weiterhin sind Substanzen (W), die aus kondensierten Phasen, wie im Falle von Wasser, Alkoholen, Aminen und deren Lösungen, einwirken, geeignet. Beschichtete Substrate können beispielsweise auch härten, indem man sie in Flüssigkeiten taucht oder mit Flüssigkeiten benetzt, die die Substanzen (W) enthalten im getauchten bzw. im mit flüssiger Substanz (w) benetzten Zustand härten. Vorzugsweise werden die Substrate zur Vermeidung von Blasen, die bei der Reaktion gebildet werden können, vor dem Tauchen strahlungsgehärtet. Die Vermeidung der Blasenbildung bei Isocyanatreaktionen mit Wasser durch Bestrahlung ist ein weiterer Vorteil der Erfindung.

Die reaktiven Substanzen (W) können sich ebenso auf Trägermaterialien befinden. Beispiele sind feuchte Substrate wie Holz, Papier, Schaumstoffe, mineralische Träger, die dann direkt mit den Verbindungen oder Mischungen I beschichtet werden können und beispielsweise nach UV-Bestrahlung zur weiteren Aushärtungsreaktion kommen. Damit sind z.B. die Beschichtung feuchter Substrate inbesondere Holz oder mineralischer Substrate z.B. Betonformplatten oder Faserzementplatten ohne Vortrocknung des Substrates möglich. Bei der Härtung durch Wasserdampf reicht der in der Luft enthaltene Wasseranteil bereits aus.

Weiterhin ist eine vollständige thermische Aushärtung ohne UV-Härtung bzw. Nachhärtung nach UV-Bestrahlung der erfindungsgemäßen Verbindungen durch Erhitzen auf 100 bis 280°C, vorzugsweise 130°C bis 200°C möglich.

Meistens werden die Verbindungen I und Mischungen I in Form von strahlungshärtbaren Zubereitungen eingesetzt, die die üblichen Hilfsmittel enthalten, also z.B. Verdicker, Entschäumer, Verlaufshilfsmittel, Farbstoffe, Füllstoffe bzw. Pigmente und, sofern erforderlich, Photoinitiatoren und Stabilisatoren (im folgenden kurz: "Zubereitungen S").

Eine Variante der Erfindung sind Zubereitungen S, die
a) 5 bis 95 Gew.-% einer Verbindung I oder einer Mischung I und
b) 95 bis 5 Gew.-% einer von den Verbindungen I verschiedenen Verbindungen mit einer radikalisch polymerisierbaren C-C-Doppelbindung (Verbindungen S) enthalten.

Bei den Verbindungen S handelt es sich häufig um sog. Reaktivverdünner oder um Bindemittel, wie sie z.B in "Chemistry & Technology of UV & EB Formulations for Coatings, Inks & Paints, Vol. 1 - 5, Ed. P. K. T. Oldring, London 1991, genannt sind.

Als Reaktivverdünner eignen sich beispielsweise vinylgruppenhaltige Monomere, insbesondere N-Vinylverbindungen, wie N-Vinylpyrrolidon, N-Vinylcaprolactam und N-Vinylformamid, weiterhin Vinylether, wie Ethyl-, Propyl-, Isopropyl-, Butyl-, Isobutyl-, tert.-Butyl-, Amyl-, 2-Ethylhexyl-, Dodecyl-, Octadecyl- und Cyclohexylvinylether, Ethylenglykolmono- und -divinylether, Di-, Tri- und Tetraethylenglykolmono- und -divinylether, Polyethylenglykoldivinylether, Ethylenglykolbutylvinylether, Triethylenglykolmethylvinylether, Polyethylenglykolmethylvinylether, Cyclohexandimethanolmono- und -divinylether, Trimethylolpropantrivinylether, Aminopropylvinylether, Diethylaminoethylvinylether und Polytetrahydrofurandivinylether, Vinylester wie Vinylacetat, - propionat, -stearat und -laurat und Vinylaromaten wie Vinyltoluol, Styrol, 2- und 4-Butylstyrol und 4-Decylstyrol sowie Acrylat- oder Methacrylatgruppen enthaltende Monomere, z. B. Hydroxyethyl(meth)acrylat, Tripropylenglykolmethylether(meth)acrylat, Cyclohexyl(meth)acrylat, 4-tert.-Butylcyclohexyl(meth)acrylat, Trimethylolpropanmonoformalacrylat, Glycerinmonoformalacrylat, 4-Tetrahydropyranylacrylat, 2-Tetrahydropyranylmethylacrylat und Tetrahydrofurfurylacrylat.

Bevorzugte Reaktivverdünner sind mono- oder polyfunktionelle Ester alpha, beta -ethylenisch ungesättigter Carbonsäuren mit aliphatischen Mono- oder Polyolen. Als Polyolkomponente kommen beispielsweise die oben genannten Di- oder Polyole, die in Zusammenhang mit den Alkoholen A genannt wurden, in Frage. Beispiele für Monoole sind übliche Alkohole bzw. deren Oxalkylierungsprodukte mit Ethylenoxid oder Propylenoxid, z.B. Methanol, Ethanol, Ethylhexanol, tertiär-Butylcyclohexanol, Tetrahhydrofurfurylalkohol, Norbornylalkohol, Laurylalkohol, Stearylalkohol, Phenoxyethylglykol, Methoxytriethylenglykol, Methoxytripropylenglykol. Die Polyalkohole können vollständig oder unvollständig bezüglich der Alkoholgruppen je Molekül mit den alpha, beta -ethylenisch ungesättigter Carbonsäuren verestert sein.
Beispiele für derartige Reaktivverdünner sind tertiär-Butylcyclohexanolacrylat, Tetrahhydrofurfurylacrylat, Norbornylacrylat, Laurylacrylat, Stearylacrylat, Phenoxyethylglykolacrylat, Methoxytriethylenglykolacrylat, Methoxytripropylenglykolacrylat (XXX weitere Monoalkohole und Acrylate XXX) Ethylenglykoldi(meth)acrylat, Ethylenglykol-(meth)acrylat, Propylenglykoldi(meth)acrylat, Propylenglykol(meth)acrylat, Butylenglykoldi(meth)acrylat, Butylenglykol(meth)acrylat, 1,6-Hexandiol-di(meth)acrylat, Diethylenglykoldi(meth)acrylat, Triethylenglykoldi(meth)-acrylat, Dipropylenglykoldi(meth)acrylat, Tripropylenglykoldi(meth)acrylat, 1,4-Cyclohexandioldi(meth)acrylat und 1,4-Bis(hydroxymethyl)cyclohexandi-(meth)acrylat, ferner Trimethylolethantri(meth)acrylat, Trimethylolethandi-(meth)acrylat, Trimethylolpropantri(meth)acrylat, Trimethylolpropandi-(meth)acrylat, Ditrimethylolpropantetra(meth)acrylat, Ditrimethylolpropantri-(meth)acrylat Pentaerythrittetra(meth)acrylat, Pentaerythrittri(meth)-acrylat, Dipentaerythrittetra(meth)acrylat, Dipentaerythritpenta(meth)acrylat. Bevorzugt sind auch die Ester ethoxylierter Polyole, z. B. die Polyacrylate oder -methacrylate von alkoxyliertem Trimethylolpropan, alkoxyliertem Ditrimethylolpropan, Glycerin, Pentaerythrit oder Dipentaerythrit.

Bei den Bindemitteln mit radikalisch polymerisierbarer C-C-Doppelbindung handelt es sich um Prepolymere, Polymere oder Oligomere vorzugsweise bis zu Molekulargewichten von 10000 wie (meth)acrylfunktionelle (Meth)acrylcopolymere, Epoxid(meth)acrylate, Polyester(meth)acrylate, Polyurethan(meth)acrylate, Polyether(methacrylate, Siikon(meth)acrylate, Melamin(meth)acrylate, ungesättigte Polyester mit Maleinsäuregruppen, ungesättigte Polyurethane mit Maleinsäuregruppen.

Eine Vielzahl der genannten Bindemittel können gegenüber Isocyanat reaktive Gruppen aufweisen, insbesondere Hydroxygruppen die z.B. unvollständig veresterten Polyolen oder in Form von β-Hydroxi(meth)acrylatgruppen in Epoxidacrylaten vorliegen. Weitere hydroxylgruppenhaltige Bindemittel sind Additionsprodukte von Isocyanatgruppen enthaltenden Komponenten mit überschüssigen Polyolen. Dadurch werden Viskosität, Topfzeit und dual cure Eigenschaften beeinflußt.

Alle Verbindungen S können auch Amingruppen bis zu einer Aminzahl von 250 mg KOH/g enthalten, z.B. durch Addition von primären oder sekundären Aminen an Doppelbindungen. Solche Amine sind vorzugsweise aliphatische Amine wie Methylamin, Dimethylamin, Ethylamin, Diethylamin, Propylamin, Dipropylamin, Butylamin, Dibutylamin, und hydroxyaliphatische Amine wie Ethanolamin, Diethanolamin, Propanolamin und Dipropanolamin.

Die Bindemittel und Reaktivverdünner können einzeln oder in Mischung eingesetzt werden.

Geeignete Füllstoffe umfassen Silikate, z. B. durch Hydrolyse von Siliciumtetrachlorid erhältliche Silikate wie Aerosil® der Fa. Degussa, Kieselerde, Talkum, Aluminiumsilikate, Magnesiumsilikate, Calciumcarbonate etc.. Geeignete Stabilisatoren umfassen typische UV-Absorber wie Oxanilide, Triazine und Benzotriazol (letztere erhältlich als Tinuvin® R-Marken der Ciba-Spezialitätenchemie) und Benzophenone. Diese können allein oder zusammen mit geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetramethyl-4-piperidyl)sebacinat, eingesetzt werden. Stabilisatoren werden üblicherweise in Mengen von 0,1 bis 5,0 Gew.-% bezogen auf die in der Zubereitung enthaltenen Komponenten mit Aktivierten Doppelbindungen eingesetzt.

Sofern die Aushärtung mittels UV-Strahlung erfolgt, enthalten die erfindungsgemäßen Zubereitungen wenigstens einen Photoinitiator, der die Polymerisation ethylenisch ungesättigter Doppelbindungen initiieren kann. Hierzu zählen Benzophenon und Benzophenonderivate, wie 4-Phenylbenzophenon und 4-Chlorobenzophenon, Michlers Keton, Acetophenonderivate, wie 1-Benzoylcyclohexan-1-ol, 2-Hydroxy-2,2-dimethylacetophenon und 2,2-Dimethoxy-2-phenylacetophenon, Benzoin und Benzoinether, wie Methyl-, Ethyl- und Butylbenzoinether, Benzilketale, wie Benzildimethylketal, 2-Methyl-1-[4-(methylthio)phenyl]-2-morpholinopropan-1-on, Anthrachinon und seine Derivate, wie Methylanthrachinon und tert.-Butylanthrachinon, Acylphosphinoxide, wie 2,4,6-Trimethylbenzoyldiphenylphosphinoxid, Ethyl-2,4,6-trimethylbenzoylphenylphosphinat und Bisacylphosphinoxide. Die vorgenannten Photoinitiatoren werden, sofern erforderlich, in Mengen von 0,05 bis 20 Gew.-%, vorzugsweise 0,1 bis 10 Gew.-% und insbesondere 0,2 bis 5 Gew.-%, bezogen auf die Verbindungen I und S der erfindungsgemäßen Zubereitungen eingesetzt.

Zur Verbesserung der Härtungsgeschwindigkeit können Amine als Coinitiatoren zugesetzt werden. Solche Amine sind üblicherweise Verbindungen wie Tributylamin, Triethanolamin, Dimethylethanolamin, Methyldiethanolamin. Diese werden in Mengen von 1 bis 10Gew% bezogen auf den Lackfestkörper eingesetzt. Ebenfalls geeignet sind Aminogrupen enthaltende Bindemittel, wie sie z.B. durch Addition von aliphatischen oder hydroxyaliphatischen primären oder sekundären Aminen, wie Methylamin, Dimethylamin, Ethylamin, Diethylamin, Propylamin, Dipropylamin, Butylamin, Dibutylamin, Ethanolamin, Diethanolamin, Propanolamin und Dipropanolamin an Acrylestermonomere und Acrylesterbindemittel vom Typ verbindungen S herstellbar sind.

Derartige aminmodifizierte Produkte können auch als Hauptbindemittel dienen und lassen sich bis zu 98 Gew% Formulierungsanteil verwenden.

Sofern die erfindungsgemäße Zubereitung mittels Elektronenstrahlung gehärtet wird, kann auf Photoinitiatoren verzichtet werden.

Bei Anwendung der Elektronenstrahlhärtung, der UV-Härtung in Kombination mit geeigneten Photoinitiatoren oder der thermischen Härtung können die erfindungsgemäßen Zubereitungen auch Pigmente enthalten.

Es ist möglich, die Zubereitungen S direkt als Beschichtungsmittel einzusetzten. In einer weiteren Ausführungsform der Erfindung werden aus den Zubereitungen S 2-Komponenten-Beschichtungsmittel hergestellt, indem man den Zubereitungen S vor der Verarbeitung eine Verbindung mit gegenüber Isocyanatgruppen reaktiven Gruppen (im folgenden kurz "Verbindungen R") zusetzt.

Sofern die Isocyanatgruppen, die die Komponenten der Zubereitungen S tragen, nicht verkappt sind, erfolgt die Vermischung der beiden Komponenten zweckmäßigerweise längstens 24 h vor dem Auftrag des Beschichtungsmittels auf den zu beschichtenden Gegenstand.

Üblicherweise beträgt das Verhältnis der Isocyanatgruppen zu den gegenüber Isocyanatgruppen reaktiven Gruppen 2:1 bis 0,5:1 bevorzugt 10:1 bis 0,7:1, besonders bevorzugt 0,9:1 bis 1,1:1.

Bei den Verbindungen R handelt es sich im allgemeinen um solche, die als A-Komponente in üblichen 2-Komponenten-Polyurethan-Beschichtungsmassen enthalten sind , also beispielsweise niedermolukulare Alkohole mit 2 bis 20 C-Atomen und 2 bis 6 OH-Gruppen oder hydroxyfunktionelle Polymere (im folgenden kurz "Polymere (A)").

Bei den Polymeren (A) handelt es sich z.B. um Polymere mit einem Gehalt an Hydroxylgruppen von 0,1 bis 20, vorzugsweise 0,5 bis 10 Gew.-%. Das zahlenmittlere Molekulargewicht Mₙ der Polymeren beträgt vorzugsweise 1000 bis 100 000, besonders bevorzugt 2000 bis 10 000. Bei den Polymeren handelt es sich bevorzugt um solche, welche zu mehr als 50 Gew.-% aus C₁- bis C₂ₒ-Alkyl(meth)acrylat, Vinylaromaten mit bis zu 20 C-Atomen, Vinylestern von bis zu 20 C-Atomen enthaltenden Carbonsäuren, Vinylhalogeniden, nicht aromatischen Kohlenwasserstoffen mit 4 bis 8 C-Atomen und 1 oder 2 Doppelbindungen, ungesättigten Nitrilen und deren Mischungen bestehen. Besonders bevorzugt sind die Polymeren, die zu mehr als 60 Gew.-% aus C₁- bis C₁₀-Alkyl- (meth)acrylaten, Styrol oder deren Mischungen bestehen.

Darüber hinaus enthalten die Polymeren (A) hydroxyfunktionelle Monomere entsprechend dem obigen Hydroxylgruppengehalt und gegebenenfalls weitere Monomere, z.B. ethylenisch ungesättigte Säuren, insbesondere Carbonsäuren, Säureanhydride oder Säureamide.

Weitere Polymere (A) sind z.B. Polyesterole, wie sie durch Kondensation von Polycarbonsäuren, insbesondere Dicarbonsäuren mit Polyolen, insbesondere Diolen erhältlich sind.

Weiterhin sind als Polymere (A) auch Polyetherole geeignet, die durch Addition von Ethylenoxid, Propylenoxid oder Butylenoxid an H-aktive Komponenten hergestellt werden. Ebenso sind Polykondensate aus Butandiol geeignet.

Bei den Polymeren (A) kann es sich natürlich auch um Verbindungen mit primären der sekundären Aminogruppen handeln.

Genannt seien z.B. sogenannte Jeffamine, d.h. mit Aminogruppen terminierte Polyetherole oder Oxazolidine.

Weiterhin sind als Verbindungen R hydroxyfunktionelle Acrylesterverbindungen geeignet wie sie z.B. durch unvollständige Veresterung von Polyolen, Polyetherolen oder Polyesterolen entstehen oder in Form von β-Hydroxi(meth)acrylatgruppen in Epoxidacrylaten vorliegen oder als Additionsprodukte von Isocyanatgruppen enthaltenden Komponenten mit überschüssigen Polyolen hergestellt werden.

Die erfindungsgemäßen Verbindungen I, Mischungen I, Zubereitungen S und 2-Komponenten Beschichtungssysteme (im folgenden kurz als "Beschichtungsmittel" bezeichnet) eignen sich als Beschichtungsmittel für unterschiedliche Substrate.

Die erfindungsgemäßen Zubereitungen erweisen sich als besonders geeignet zum Beschichten von Substraten wie Holz, Holzwerkstoffen, Papier, Textil, Leder, Vlies, Kunststoffoberflächen, Glas, Keramik, mineralischen Baustoffen, wie Zement-Formsteine und Faserzementplatten, und insbesondere von Metallen oder entsprechende vorbeschichtete Substraten.

Demnach betrifft die vorliegende Erfindung auch ein Verfahren zum Beschichten von Substraten, insbesondere von Metallen oder beschichteten Metallen, wie sie z.B. auch im Fahrzeugkarosserieoder Coil Coating-Bereich eingesetzt werden, sowie die durch dieses Verfahren erhältlichen beschichteten Substrate. Die Beschichtung der Substrate erfolgt in der Regel dadurch, dass man wenigstens eine erfindungsgemäße, strahlungshärtbare Zubereitung auf das zu beschichtende Substrat in der gewünschten Stärke aufbringt und gegebenenfalls vorhandenes Lösungsmittel entfernt. Dieser Vorgang kann, sofern gewünscht, ein- oder mehrfach wiederholt werden. Das Aufbringen der strahlungshärtbaren Zubereitungen auf das Substrat erfolgt in bekannter Weise, z. B. durch Tauchen, Spritzen, Spachteln, Rakeln, Bürsten, Rollen, Walzen oder Gießen.

Erfindungsgemäß beschichtete Folien z.B. aus Papier oder Kunststoff können ebenfalls durch Kaschieren, gegebenenfalls mit Hilfe eines Klebers. Dabei kann der Kleber sowohl auf die beschichteten Folien als auch auf das Substrat aufgebracht werden. Die entweder photochemisch oder thermisch teilgehärteten Filme können vor, während oder nach der Applikation verformt werden. Damit werden Folienbeschichtungen auf nichtplanen Substraten oder die Verformung der beschichteten Substrate möglich.

Die Beschichtungsstärke liegt in der Regel im Bereich von 3 bis 3000 g/m² und vorzugsweise 10 bis 2900 g/m². Ein Vorteil der erfindungsgemäßen Beschichtungen, insbesondere aufgrund der niedrigviskos und lösemittelfrei verarbeitbaren Flüssigkeiten, ist die einfache Herstellung dicker,luftblasenfreier Schichten in einem Arbeitsgang. Das Aufbringen kann sowohl bei Raumtemperatur als auch bei erhöhter Temperatur, vorzugsweise jedoch nicht oberhalb 200°C erfolgen. Im Falle dicker Schichten über 100µm, die thermisch vorgehärtet werden, werden zur Vermeidung von Luftblasen Härtungstemperaturen unter 100°C, ganz besonders unter 60°C bevorzugt.

Im allgemeinen härtet man anschließend die Beschichtungen sowohl durch Bestrahlung mit energiereicher Strahlung als auch dadurch, daß man die Isocyanatgruppen mit Luftfeuchtigkeit oder Verbindungen R reagieren läßt. Diese Vorgehensweise wird als dual cure Verfahren bezeichnet.

Im Unterschied zur Strahlenhärtung, die innerhalb weniger Sekunden oder Bruchteilen von Sekunden abläuft, verläuft die Isocyanathärtung in der Regel langsam, d.h. sie ist bei Raumtemperatur erst oftmals nach Tagen abgeschlossen. Sie kann aber auch durch Auswahl geeigneter Katalysatoren, bei erhöhter Temperatur, bevorzugt bis 200°C oder durch Zusatz geeigneter reaktiver Reaktionspartner beschleunigt werden. Bei Härtungstemperaturen oberhalb 120°C können die reaktiven Doppelbindungen ebenfalls ohne Zusatz thermischer Initiatoren reagieren und zur Härtung beitragen, so daß auf eine zusätzliche Bestrahlung verzichtet werden kann. Härtungstemperaturen zur Polymerisation der Doppelbindungen ohne Bestrahlung können durch Zusatz thermisch radikalbildender Polymerisationsinitiatoren wie z.B. organischer Peroxid- oder Azoverbindungen gegebenenfalls in Kombination mit Beschleunigern auf Basis von Kobalt-Verbindungen bzw. Aminen bis unter Raumtemperatur gesenkt werden.

Gegebenenfalls kann, wenn mehrere Schichten des Beschichtungsmittels übereinander aufgetragen werden, nach jedem Beschichtungsvorgang eine Strahlungshärtung erfolgen.

Die Strahlungshärtung erfolgt durch Einwirkung energiereicher Strahlung, also UV-Strahlung oder Tageslicht, vorzugsweise Licht der Wellenlänge 250 bis 600 nm oder durch Bestrahlung mit energiereichen Elektronen (Elektronenstrahlung; 150 bis 300 keV). Als Strahlungsquellen dienen beispielsweise Hochdruckquecksilberdampflampen, Laser, gepulste Lampen (Blitzlicht), Halogenlampen oder Excimerstrahler. Die üblicherweise zur Vernetzung ausreichende Strahlungsdosis bei UV-Härtung liegt im Bereich von 80 bis 3000 mJ/cm². Unter Verwendung geeigneter langwellig absorberender Fotoinitiatoren insbesondere mit Acylphosphinoxiden 2,4,6-Trimethylbenzoyldiphenylphosphinoxid, Ethyl-2,4,6-benzoylphenylphosphinat und Bisacylphosphinoxide, können Beschichtungen auch unter tageslichtähnlichen Lichtquellen oder an Sonnenlicht aushärten.

Besonders effizient hinsichtlich Energiebedarf, Fotoinitiatorbedarf und Oberflächenqualität, insbesondere der Kratzbeständigkeit und Chemikalienbeständigkeit, kann die Bestrahlung gegebenenfalls auch unter Ausschluß von Sauerstoff, z. B. unter Inertgas-Atmosphäre, oder unter Atmosphären mit verminderten Sauerstoffgehalten unter 17 Gew.-% durchgeführt werden. Als Inertgase eignen sich vorzugsweise Stickstoff, Edelgase, Kohlendioxid, oder Verbrennungsgase. Desweiteren kann die Bestrahlung erfolgen, indem die Beschichtungsmasse mit transparenten Medien abgedeckt wird. Transparente Medien sind z.B. Kunstofffolien, Glas oder Flüssigkeiten, z.B. Wasser.

In einem bevorzugten Verfahren erfolgt die Aushärtung kontinuierlich, indem man das mit der erfindungsgemäßen Zubereitung behandelte Substrat mit konstanter Geschwindigkeit an einer Strahlungsquelle vorbeiführt. Hierfür ist es erforderlich, dass die Aushärtungsgeschwindigkeit der erfindungsgemäßen Zubereitung ausreichend hoch ist.

Diesen unterschiedlichen zeitlichen Verlauf der Härtung kann man sich insbesondere dann zu Nutze machen, wenn sich an die Beschichtung des Gegenstandes noch ein Verarbeitungsschritt anschließt, bei dem die Filmoberfläche in direkten Kontakt mit einem anderen Gegenstand tritt oder mechanisch bearbeitet wird.

Dabei geht man so vor, daß man
Ia. in Schritt Ia einen Gegenstand mit einem Film des Beschichtungsmittels beschichtet,
IIa. in Schritt IIa den Film des Beschichtungsmittels mit energiereicher Strahlung bestrahlt, wobei der Film vorgehärtet wird,
IIIa. in Schritt IIIa den Gegenstand, der mit dem vorgehärteten Film des Beschichtungsmittels beschichtet ist, bearbeitet, insbesondere verformt, mit einer weiteren oder mehreren Beschichtungen überschichtet oder die Oberfläche des vorgehärteten Films mit einem anderen Gegenstand in Kontakt bringt und
IVa. in Schritt IVa den vorgehärteten Film des Beschichtungsmittels, mit dem der bearbeitete Gegenstand beschichtet ist, durch Isocyanathärtung endhärtet.
oder nach einem 2. Verfahren, bei dem man
Ib. in Schritt Ib einen Gegenstand mit einem Film des Beschichtungsmittels beschichtet,
IIb. in Schritt IIb den Film des Beschichtungsmittels, mit dem der Gegenstand beschichtet ist, vorhärtet, indem man die Isocyanatgruppen in einer Polyadditionsreaktion reagieren läßt,
IIIb. in Schritt IIIb den Gegenstand, der mit dem vorgehärteten Film des Beschichtungsmittels beschichtet ist, bearbeitet, insbesondere verformt, mit einer weiteren oder mehreren Beschichtungen überschichtet oder die Oberfläche des vorgehärteten Films mit einem anderen Gegenstand in Kontakt bringt und
IVb. in Schritt IVb den vorgehärteten Film des Beschichtungsmittels mit energiereicher Strahlung bestrahlt, wobei der Film endgehärtet wird.

Der Vorteil dieser Verfahren liegt darin, daß man die beschichteten Gegenstände unmittelbar im Anschluß an Schritt IIa bzw. IIb weiterverarbeiten kann, weil die Oberfläche klebfrei oder auch klebrig eingestellt werden kann. Der vorgehärtete Film ist noch so flexibel und dehnbar, daß der Gegenstand verformt werden kann, ohne daß der Film dabei abplatzt oder reißt. Im Falle der Klebfreiheit ist insbesondere eine direkte mechanische Bearbeitung wie Verformen oder Schleifen ohne Verklebungen des Schleifpapiers möglich.

Selbst wenn keine Verformung des Gegenstandes vorgesehen ist, kann sich das sog. dual cure Verfahren vorteilhaft erweisen, denn die mit dem vorgehärteten Film versehenen Gegenstände können besonders einfach transportiert und gelagert werden, z.B. in Stapeln. Darüberhinaus bietet das dual cure Verfahren den Vorteil, daß die Beschichtungsmassen in Dunkelbereichen (Bereiche, die für die Strahlung nicht zugänglich sind) chemisch nachhärten können und somit noch ausreichende Materialeigenschaften unabhängig von der Bestrahlung erreicht werden. Desweiteren härten Spritznebelniederschläge kleb- und emissionsfrei aus. Besonders geeignet werden diese Beschichtungsmassen damit auch für den Einsatz als Dichtungsmassen mit schneller Aushärtung an beleuchtbaren Stellen und einer Nachhärtung in Dunkelbereichen.

In Substraten aufgesaugtes Material, oder allgemein bei Verwendung saugfähiger, poröser Untergründe wie Holz, Papier, mineralischer Substrate, Textilien, Leder, Schäumen, härtet weggeschlagenes Material aus, so daß emittierbare, migrierbare oder extrahierbare Anteile vermieden werden.

Im Anschluß an Schritt III wird häufig Schritt IVa so ausgeführt, daß die beschichteten Gegenstände noch einige Tage bei Raumtemperatur oder erhöhter Temperatur an der Luft gelagert werden, um den Härtungsprozeß zu beschleunigen. Während dieser Zeit reagieren, wie zuvor beschrieben, die Isocyanatgruppen mit Luftfeuchtigkeit oder ggf. der A-Komponente, wobei sich die Netzwerkdichte erhöht und der Film seine endgültigen Gebrauchseigenschaften erhält.

Die Verbindungen I, Mischungen I oder eine Zubereitung S sind insbesondere als Gießharz, Spachtelmasse, Dichtungsmasse, Lötstopplack, Photoresistharz, Stereolithographieharz, Druckfarbe, Klebstoff, Dentalmasse, zur Herstellung photopolymerer Druckplatten, als Harz für Verbundwerkstoffe oder als Lack für die Fahrzeugbeschichtung insbesondere zur Beschichtung von Karosserieteilen einsetzbar.

Für die Anwendungen als Photoresistharz, Lötstopplack, Stereolithographieharz und photopolymerer Druckplatte ist die Eigenschaft der ortsdefinierten Aushärtbarkeit mittels Bestrahlung über Masken oder mittels punktförmigen Strahlenbündel z.B. Laserstrahlung, entscheidend. Damit kann in einem der abbildenden Strahlenhärtung nachgeschalteten Auswaschprozeß mit Lösemitteln oder wässrigen Waschflüssigkeiten ein Relief erzeugt werden. Durch thermische Nachhärtung der photopolymerisierten Massen werden höhere mechanische und chemische Beständigkeiten erhalten, die für den Einsatz z.B. als Druckplatte, Leiterplatte oder als sonstiges Formteil benötigt werden.

### Experimenteller Teil

### 1. Herstellung der Verbindungen I

### 1.1. Herstellung der Urethan- und Allophanatgruppen enthaltenden Polyisocyanato-acrylate 1 - 9 und des Vergleichsbeispiels 1 (V1) aus HDI und ungesättigten Monoalkoholen

Hexamethylendiisocyanat (HDI) wurde unter Stickstoffbedeckung vorgelegt und die in Tabelle 1 genannte Menge an stabilisierter OH-Komponente zugesetzt. Man erwärmte die Mischung auf 80°C und gab 200 Gew. ppm (bezogen auf Diisocyanat) des Katalysators N,N,N-Trimethyl-N-(2-hydroxypropyl)-ammonium-2-ethylhexanoat zu. Die Temperatur erhöhte sich langsam auf 120°C. Man ließ bei dieser Temperatur reagieren und stoppte die Reaktion bei dem in Tabelle 1 genannten NCO-Gehalt der Mischung durch Zugabe von 250 Gew. ppm (bezogen auf Diisocyanat) Di-2-(Ethylhexyl)-phosphat. Das Reaktionsgemisch wurde anschließend im Dünnschichtverdampfer bei 135°C und 2.5 mbar von nicht umgesetztem HDI befreit.

Daten zu den Endprodukten stehen in Tabelle 1.

### 1.2. Herstellung des Urethan- und Allophanatgruppen enthaltenden Polyisocyanato-acrylats 10

Hexamethylendiisocyanat (HDI) wurde unter Stickstoffbedeckung vorgelegt und die in Tabelle 1 genannte Menge an stabilisiertem 3-(Acryloyloxy)-2-hydroxypropylmethacrylat zugesetzt. Man erwärmte die Mischung auf 80°C und gab 500 Gew. ppm (bezogen auf Diisocyanat) des Katalysators Zink-Acetylacetonat zu. Die Temperatur wurde anschließend langsam auf 120°C erhöht. Man ließ bei dieser Temperatur reagieren und stoppte die Reaktion bei dem in Tabelle 1 genannten NCO-Gehalt der Mischung durch Zugabe von 550 Gew. ppm (bezogen auf Diisocyanat) Di-2-(Ethylhexyl)-phosphat. Das Reaktionsgemisch wurde anschließend im Dünnschichtverdampfer bei 135°C und 2.5 mbar von nicht umgesetztem HDI befreit.

Daten zum Endprodukt stehen in Tabelle 1.

### 1.3. Herstellung der Urethan- und Allophanatgruppen enthaltenden Polyisocyanato-acrylate 11 und 12 aus IPDI bzw. 1,3-BIC und ungesättigten Monoalkoholen

Die Diisocyanate wurden unter Stickstoffbedeckung vorgelegt und die in Tabelle 1 genannte Menge an stabilisierter OH-Komponente zugesetzt. Man erwärmte die Mischung auf 100°C und gab 200 Gew. ppm (bezogen auf Diisocyanat) des Katalysators N,N,N-Trimethyl-N-(2-hydroxypropyl)-ammonium-2-ethylhexanoat zu. Die Temperatur erhöhte sich langsam auf 120 °C. Man ließ bei dieser Temperatur reagieren und stoppte die Reaktion bei dem in Tabelle 1 genannten NCO-Gehalt der Mischung durch Zugabe von 250 Gew. ppm (bezogen auf Diisocyanat) Di-2-(Ethylhexyl)-phosphat. Das Reaktionsgemisch wurde anschließend im Dünnschichtverdampfer bei 135°C und 2.5 mbar von nicht umgesetztem Isocyanat befreit.

Daten zu den Endprodukten stehen in Tabelle 1.

### 1.4. Vergleichsversuche, Herstellung von Urethanacrylaten analog zu EP 549 116 Hydroxyethylacrylat aus HDI-Polyisocyanat und HEA

625 g (1 mol) HDI-Polyisocyanat mit einer mittleren Funktionalität von ca. 3.5 und einem NCO-Gehalt von 22.0 Gew.% (BASONAT®HI 100, BASF AG) wurden unter Stickstoffbedeckung vorgelegt, mit 200 ppm Dibutylzinn-dilaurat versetzt und auf 55°C erwärmt. Innerhalb 15 min wurden die in Tabelle 2 angegebenen Mengen an Hydroxyethylacrylat (stabilisiert) zugegeben und der Ansatz langsam auf 80°C erwärmt. Anschließend wurde noch eine Stunde bei 80°C nachgerührt.

Daten zu den Beschichungsmitteln des Standes der Technik finden sich in Tabelle 2

Abkürzungen:
- HDI =: Hexamethylendiisocyanat
- IPDI =: Isophorondiisocyanat
- 1,3-BIC =: 1,3-Bis (isocyanatomethyl) cyclohexan
- HEA =: Hydroxyethylacrylat
- HPA =: Hydroxypropylacrylat
- HEMA =: Hydroxyethylmethacrylat
- GAMA =: 3-(Acryloyloxy)-2-hydroxypropylmethacrylat
- DBTL =: Dibutylzinn-dilaurat

### 2. Herstellung und Prüfung von Lackformulierungen aus den erfindungsgemäßen Polyisocyanaten als 1-Komponenten-Beschichtungssysteme

Die erfindungsgemäßen Produkte bzw. Vergleichsprodukte wurden, falls sie eine Viskosität von mehr als 500 mPas aufwiesen mit Butylacetat (BuAc) auf 500 mPas verdünnt. Die Proben wurden mit Filmziehrahmen auf Glas oder Blech aufgezogen.

Die erfindungsgemäßen Produkte bzw. Vergleichsprodukte wurden auf unterschiedliche Weise gehärtet und geprüft:

### UV-Bes trahlung:

Die gegebenenfalls bei Raumtemperatur vom Lösemittel abgelüfteten Filme werden 5 mal bei 10m/min Bandgeschwindigkeit unter einem IST-Quecksilberhochdruckstrahler (120W/cm) bestrahlt.

### Feuchtigkeitshärtung:

Die Filme werden über mehrere Tage hinweg bei Raumtemperatur und 50%iger Luftfeuchtigkeit gelagert.

Tabelle 3 beschreibt das Ergebnis nach Härtung nur mit Luftfeuchtigkeit

Tabelle 4 beschreibt das Ergebnis nach Härtung mit Luftfeuchtigkeit und UV-Härtung

### Prüfmethoden:

- Pendeldämpfung (PD, in Anzahl Schwingungen): Lack auf Glas als Substrat (DIN 53157) bei einer Lackschichtdicke, trocken, von ca. 30 µm im Fall der Luf thärtung und mit ca. 50 µm im Fall der UV-Härtung.
- Erichsentiefung (ET; DIN 53156, in mm Tiefung): Lack auf Bonderblech 132 (Maße 190 x 105 x 1mm von Fa. Chemetall). Lackschichtdicke 25 bis 30 µm.
- Haftung mit Gitterschnitt (HmG; DIN 53151, in Noten): Lack auf Bonderblech 132 (Maße 190 x 105 x 1mm von Fa. Chemetall). Lackschichtdicke, trocken, 25 bis 30 µm. Die Beurteilung erfolgt mit Hilfe einer Notenskala von 0 bis 5 (0 = beste Note)

### 3. Zweikomponenten-Lacksysteme unter Zusatz von NCO-reaktiven Verbindungen

Das erfindungsgemäße Produkt Nr. 6 (s. Tabellen 1 und 5) wurde mit dem hydroxifunktionellen Vinylpolymerisat (Lumitol® H 136, BASF) zum einen entsprechend dem stöchiometrischen OH/NCO-Verhältnis, zum anderen in gleichen Mengenverhältnissen zum Vergleichsversuch gemischt. Als Vergleich wurde ein Klarlack auf Basis desselben Acrylatharzes in Kombination mit dem Polyisocyanathärter (Basonat® P LR 8901, BASF) geprüft. Die Einstellung auf eine Applikationsviskosität von 20 s (DIN 53 211 Becher 4 mm Auslaufdüse) erfolgte mit Butylacetat.

Mit einem Filmziehrahmen wurden auf Glasplatten Beschichtungen mit einer Naßfilmdicke von 200 ∝m aufgetragen. Die so erhaltenen Klarlacke wurden unter Normklima gehärtet.

Die bei Raumtemperatur vom Lösemittel abgelüfteten Filme werden 9 mal bei 15 m/min Bandgeschwindigkeit unter einem IST-Quecksilberhochdruckstrahler (120W/cm) bestrahlt.

Die erhaltenen Lackeigenschaf ten sind in Tabelle 6 und 7 zusammengefaßt.

**Tabelle 1:**

| Reaktionsprodukte aus Isocyanaten und ungesättigten Monoalkoholen | | | | | | |
|---|---|---|---|---|---|---|
| Produkt Nr. | Isocyanat | Monoalkohol | Menge bez. Isocyanat (mol.-%) | NCO-Gehalt der Mischung (Gew.-%) | Nco-Gehalt nach Destillation (Gew.-%) | Viskosität bei 23°C (mPas) |
| 1 | HDI | HEA | 5 | 40.8 | 20.1 | 520 |
| 2 | HDI | HEA | 10 | 39.0 | 18.5 | 310 |
| 3 | HDI | HEA | 15 | 35.6 | 17.4 | 290 |
| 4 | HDI | HEA | 20 | 33.3 | 16.7 | 260 |
| 5 | HDI | HEA | 20 | 26.7 | 15.8 | 1790 |
| 6 | HDI | HEA | 40 | 21.6 | 13.5 | 810 |
| 7 | HDI | HEA | 50 | 16.8 | 11.8 | 1640 |
| 8 | HDI | HPA | 20 | 32.8 | 16.0 | 345 |
| 9 | HDI | HEMA | 30 | 23.2 | 14.2 | 1290 |
| 10 | HDI | GAMA | 20 | 36.8 | 12.8 | 890 |
| 11*) | IPDI | HEA | 20 | 30.1 | - | 5680 |
| 12 | 1,3-BIC | HEA | 20 | 30.4 | 15.0 | 27500 |
| V1 (Vergleich) | HDI | Allylalkohol | 20 | 34.0 | 17.8 | 240 |

| | | | | | | |
|---|---|---|---|---|---|---|
| *) : Das Reaktionsgemisch wurde nicht destilliert | | | | | | |

**Tabelle 2:**

| Urethanacrylate als Vergleichsprodukte | | | | | |
|---|---|---|---|---|---|
| Vergleich Nr. | Menge HEA (g) | Mol HEA | HEA-Anteil (Gew.-%) | NCO-Gehalt (Gew.-%) | Viskosität 23°C (mPas) |
| V2 | 75.6 | 0.65 | 10.8 | 15.3 | 12220 |
| V3 | 116.0 | 1.0 | 15.7 | 13.2 | 24200 |
| V4 | 232.0 | 2.0 | 27.1 | 6.2 | 220000 |

V2 wurde gemäß den Angaben in der EP 549 116 hergestellt, die Produkte V3 und V4 wurden auf einen höheren Doppelbindungsanteil ausgelegt.

Der Anteil an Acrylat ist bei diesen Produkten im Vergleich zu den erfindungsgemäßen Verbindungen I (Anteil beim einfachsten Allophanat = 25.7 Gew.%) geringer, trotzdem sind die viskositäten bereits sehr hoch.

Erhöhung der Kratzfestigkeit in Vergleich zu einem Autoklarlack

K1) 50 Teile eines Allophanatadduktes aus HEA und HDI mit 12,1% NCO und einer Viskosität von 1Pas mit dem Hilfsstoffsystem aus 1Teil einer 1%igen Dibutylzinndilauratlösung in Butylacetat, 2Teilen einer Mischung aus Irgacure 184 (Ciba Spezialitätenchemie) und 0,5 Teilen Lucirin TPO (BASF AG) (Mengenverhältnis 8,75: 1,25 ), 0,5 Teilen Tinuvin 292 (Ciba S.) und 0,75 Teilen Tinuvin 400 (Ciba S.) werden auf eine Glasplatte mit 50 µm Schichtdicke aufgetragen. Der Film wird zweimal unter einer UV-Quecksilberhochdrucklampe (120W/cm) mit 5m/min Bandgeschwindigkeit bestrahlt. Anschließend wird 30 Minuten bei 130°C erwärmt.

Ein mit Siliciumcarbid belegter Schwamm wird unter Belastung eines 500g schweren Hammers über den erkalteten Lackfilm bewegt. Der Glanzverlust gemessen bei 60° nach unterschiedlicher Hubzahl wurde bestimmt als Maß für die Kratzempfindlichkeit.

K2) Analog K1) wurden aus 44,7 Teilen des Allophanatacrylates mit 6,54 Teilen von 1,2-Propandiol sowie dem Hilfsstoffsystem von 1) ein Film hergestellt und geprüft.

K3) Analog K1) wurden aus 39,4 Teilen des Allophanatacrylates mit 10,6 Teilen eines Polyetherols aus dem Addukt aus 1 Mol Trimethylolpropan mit 3 Mol Ethylenoxid (Lupranol VP 9236) sowie dem Hilfsstoffsystem von 1) ein Film hergestellt und geprüft.

| Beispiel Zusammensetzung | K1 | K2 | K3 |
|---|---|---|---|
| Allophanatacrylat (12,1 % NCO, Visk. 1 Pas) | 50 | 44,7 | 39,4 |
| 1,2 Propandiol | | 6,54 | |
| Lupranol VP 9236 | | | 10,6 |

Als Vergleich diente ein lösemittelhaltiger Zweikomponenten-Polyurethanlack (2K PU) aus der Automobilserienlackierung (BASF Coatings AG) , der analog bei 130°/30 Minuten eingebrannt wurde.

| | Anzahl Doppelhübe | | |
|---|---|---|---|
| Glanz 60° | 0 | 10 | 50 |
| Beispiel K1 | 90 | 70 | 63 |
| Beispiel K2 | 96 | 59 | 44 |
| Beispiel K3 | 95 | 84 | 73 |
| 2K PU | 89 | 36 | 14 |

Die Isocyanatoacrylatlacke zeigen einen deutlich niedrigeren Glanzverlust und damit eine höhere Kratzbeständigkeit. Sie sind für die Automobillackierung geeignet. Beispiel K1 hat den zusätzlichen Vorteil der Verarbeitbarkeit als Einkomponentenlack.

### Grundierung von Holz zur Reduzierung extrahierbarer Bestandteile

| G1) UV-Walzlackierung von Buchefurnier mit konventionelllem UV-Lack | | |
|---|---|---|
| Lack 1(Vergleich) | 60 Teile | Polyesteracrylat (Laromer PE 56F) |
| | 40 Teile | Tripropylenglykoldiacrylat(TPGDA) |
| | 4 Teile | Irgacure 184 (Fotoinitiator der Fa. Ciba Spezialitätenchemie) |
| | 1 Teil | Benzophenon |
| | 1 Teil | CAB 551-001 (Celluloseacetobutyrat der Fa. Kodak) |
| | viskosität: | 1,2 Pas |

Lack 1 wird als Grundierung mit 26 g/m² auf Buchefurnier aufgetragen und bei 10 m/min UV-gehärtet, anschließend werden weitere 24g/m² aufgetragen und bei 5 m/min mit UV endgehärtet.

Zur Bestimmung der extrahierbaren Anteile (3 Tage nach Applikation) werden 15 cm² des beschichteten Furniers zerkleinert und mit 10 ml Methylenchlorid (überdeckend) in einer Duranampulle für 1h bei 40°C extrahiert.

An extrahierbaren Acrylatbestandteilen wurde TPGDA mittels GC/MS zu 1330 mg /m² Furnier bestimmt.

| G2) UV-Walzlackierung von Buchefurnier mit erfindungsgemäßem Lack | | |
|---|---|---|
| Lack 2 | 100 Teile | Allophanataddukt aus HDI und HEA NCO-Wert 12,8%, Viskosität 1 Pas |
| | 4 Teile | Irgacure 184 |
| | Viskosität: | 1,6 Pas |

Lack 2 wird als Grundierung mit 25 g/m² auf Buchefurnier aufgetragen und bei 2 m/min zu einer klebfreien Schicht UV-gehärtet, anschließend werden 23g/m² von Lack 1 aufgetragen und zweimal bei 2 m/min mit UV-gehärtet.

Extrahierbare Anteile an Acrylaten liegen unter der Nachweisgrenze (< 10 mg/m²).

### Beispiel zur UV-Walzlackierung und Verformung

Lack2) (s. K2) wird auf Kirschbaumfurnier mit 80g/m² aufgetragen und zweimal bei 2m/min UV gehärtet.

Das Furnier wird mit einer Leimharzfolie auf einen profilierten Träger bei 120°C 100 kp /cm² mit einer profilierten Gegenform aufgepresst. Die Verformbarkeit der Lackschicht ist für die Verformung des Furnieres ausreichend und reißt erst mit Beschädigung des Furnieres.

### Beispiel zur Herstellung dickschichtiger verformbarer und nachhärtbarer Filme

20,76 Teile eines Allophanatadduktes aus HEA und HDI mit 12,1% NCO und einer Viskosität von 1 Pas werden mit 33,33 Teilen Luprenal VP 9236 (BASF, ethoxiliertes Trimethylolpropan, OH-Zahl 605 mg KOH/g), 0,91 Teile Propylenglykol und 0,5 Teile einer 1 %igen Lösung von Dibutylzinndilaurat in Butylacetat sowie 1 Teil einer Mischung aus Irgacure 184 (Ciba Spezialitätenchemie) und Lucirin TPO (BASF) (Gewichtsverhältnis 3,5:0,5) luftblasenfrei abgemischt. Der Lack wird in eine Polyethylenschale zu einem 3 mm hohen Film gegossen. Zur Aushärtung wird unter Lichtausschluß 1 Stunde bei Raumtemperatur und 30 min bei 60°C sowie 24 Stunden bei Raumtemperatur gelagert. Es entsteht ein elastomerer, transparenter und blasenfreier Dickschichtfilm, der auch unter Erwärmen auf 130°C keine Blasen erzeugt und nicht fließt. Nach UV-Bestrahlung bei 2 * 5 m/min Bandgeschwindigkeit unter einer 120 W/cm Quecksilberhochdrucklampe erhärtet der Elastomerfilm zu einem hochbeständigen, transparenten Duroplastkörper.

### Herstellung eines hochdeckenden, rot pigmentierten Lackfilmes

62,4 Teile eines Allophanatadduktes aus HEA und HDI mit 12,1% NCO und einer Viskosität von 1Pas werden mit 3 Teilen Butylacetat, 9 Teile Disperbyk 163 (Fa. Byk) und 30 Teilen Irgazin DPP Rot BO mittels 150 Teilen Zirkonkugeln in einem Mischapparat der Skandex Disperser über 1 Stunde zu einer Pigmentpaste dispergiert und gesiebt. Zu 50 Teilen dieser Paste werden 3,6 Teile einer Mischung aus Trimethylolpropan und Propylenglykol (Gewichtsverhältnis 2 : 1), 0,13 Teile Byk 307 (Fa. Byk) und 1,07 Teile einer 1 %igen Lösung von Dibutylzinndilaurat in Butylacetat sowie 2 Teile einer Mischung aus Irgacure 184 (Ciba Spezialitätenchemie) und Lucirin TPO (BASF) (Gewichtsverhältnis 3,5:0,5) abgemischt. Mit einem Rakel werden 50 µm Film auf eine Glasplatte aufgezogen und 15 min bei 80°C erwärmt.

Nach UV-Bestrahlung bei 2* 5 m/min Bandgeschwindigkeit unter einer 120 W/cm Quecksilberhochdrucklampe erhärtet die Filmoberfläche ohne Ausbildung von Oberflächenstörungen, diese entstehen jedoch wenn der Film ohne Erwärmen unmittelbar nach dem Auftrag bestrahlt wird.

### Herstellung eines hochdeckenden, rot pigmentierten, tiefziehfähigen und inert belichtbaren Lackfolie

Der rot pigmentierte Lack desletzten Beispiels, allerdings mit auf 0,25 Teile deutlich verringertem Fotoinitiatoranteil des obigen Beispiels wird auf eine tiefziehfähige Polypropylenfolie in 50 µm Dicke aufgetragen und 15 min bei 80°C erwärmt.

Dieser Folienverbund kann faltenfrei über eine nichtplane Oberfläche, z.B. einer Tischplattenecke, gezogen und aufgepresst werden. Die Tiefziehfähgigkeit bleibt erhalten wenn die Folie lichtgeschützt gelagert wird. Zur Erhöhung der Klebfestigkeit kann eine zusätzliche Klebstoffschicht entweder auf das Substrat oder auf der Lackfolie oder auch beidseitig eingesetzt werden. Nach UV-Bestrahlung bei 10 m/min Bandgeschwindigkeit unter einer 120 W/cm Quecksilberhochdrucklampe erhärtet die Filmoberfläche zu einer hochbeständigen, wetterfesten und kratzfesten Lackierung.

Eine weitere Verbesserung vor allem auch hinsichtlich dem optischen Erscheinungsbild und Beständigkeitseigenschaf ten wird erreicht, wenn als verformbare Decklackschicht zusätzlich ein Klarlack entsprechend dem Beispiel zur Herstellung dickschichtiger verformbarer und nachhärtbarer Filme zwischen Polypropylenfolie und Farbschicht aufgetragen wird.

### Beispiel zur Herstellung eines photostrukturierten Reliefs

50 Teile eines Allophanatadduktes aus HEA und HDI mit 12,1% NCO, Viskosität 1 Pas, werden mit 0,5 Teilen einer 1 %igen Lösung von Dibutylzinndilaurat in Butylacetat sowie 1 Teil einer Mischung aus Irgacure 184 (Ciba Spezialitätenchemie) und Lucirin TPO (BASF) (Gewichtsverhältnis 3,5:0,5) abgemischt. Die Mischung wird auf eine Polyesterfolie zu 40 µm Schichtdicke aufgetragen, mit einer Bildmaske abgedeckt und mit 5m/min Bandgeschwindigkeit UV-belichtet. Im Anschluß wird mit Aceton ungehärtetes Material abgewaschen. Belichtete Flächen bleiben als Relief zurück. Das Relief härtet beim Lagern an Luft mit Luftfeuchtigkeit nach und wird härter und quellbeständiger.

## Patentansprüche

1. Verbindungen mit Isocyanatgruppen oder verkappten Isocyanatgruppen, Allophanatgruppen und radikalisch polymerisierbaren C-C-Doppelbindungen, wobei die C-C-Doppelbindungen durch eine direkt daran gebundene Carbonylgruppe oder ein O-Atom in Etherfunktion aktiviert sind (Aktivierte Doppelbindungen), abgeleitet von aliphatischen oder alicyclischen Polyisocyanaten und Alkoholen A, die neben der Alkoholgruppe noch eine Aktivierte Doppelbindung tragen.

2. Verbindungen nach Anspruch 1 der Formel I
OCN-R¹-(-R²-C(O)-R²-R¹-)ₙ-NCO I
in der
n eine ganze Zahl von 1 bis 10
R¹ eine divalente aliphatische oder alicyclische C₂- bis C₂₀-Kohlenwasserstoffeinheit
R² in jeder Wiederholungseinheit einmal für -NH- steht und einmal für N-C(O)-R³, wobei R³ für einen von einem Alkohol A durch Abstraktion des H-Atoms von der alkoholischen Hydroxylgruppe abgeleiteten Rest steht, wobei der Alkohol A als funktionelle Gruppe neben der Alkoholgruppe noch eine Aktivierte Doppelbindung trägt,
bedeutet.

3. Verbindungen nach Anspruch 1 oder 2, wobei es sich bei dem Alkohol A um einen Ester aus einem aliphatischen oder aromatischen Polyol und Acryl- oder Methacrylsäure, um ein Amid aus einem Aminoalkohol und Acryl- oder Methacrylsäure oder einen Vinylether, abgeleitet von einem aliphatischen oder aromatischen Polyol handelt.

4. Verbindungen nach Anspruch 3, wobei es sich bei dem aliphatischen oder aromatischen Polyol um ein Di-, Tri- oder Tetrol mit 2 bis 20 C-Atomen oder um ein Polyether- oder Polyesterpolyol oder um ein Polyacrylat-polyol mit einer mittleren OH-Funktionalität von 2 bis 10 handelt.

5. Verbindungen nach den Ansprüchen 1 bis 4, wobei der Rest R³ von Hydroxyethylacrylat, Hydroxypropylacrylat, Hydroxybutylacrylat, Hydroxyethylmethacrylat oder 3-(Acryloyloxy)-2-hydroxypropylmethacrylat abgeleitet ist.

6. Verbindungen nach den Ansprüchen 1 bis 5, wobei es sich bei dem Isocyanat um Hexamethylendiisocyanat Isophorondiisocyanat, Tetramethylxylylendiisocyanat, Di(isocyanatocyclohexyl)methan oder 1,3-Bis-(isocyanatomethyl)-cyclohexan handelt.

7. Mischungen, enthaltend
a1) 1 bis 100 Gew.-% Verbindungen gemäß Anspruch 1
a2) 0 bis 99 Gew.-% einer sonstigen Verbindung, die neben einer oder mehreren Isocyanatgruppen noch eine Gruppe, ausgewählt aus der Gruppe der Urethan-, Harnstoff-, Biuret-, Allophanat-, Cabodiimid-, Uretonimin-, Uretdionund Isocyanuratgruppen enthält.

8. Mischungen nach Anspruch 7, wobei der Gehalt an Aktivierten Doppelbindungen, bezogen auf die Gesamtmenge der Mischungskomponenten a1 und a2, 0,02 bis 20 Gew.-% beträgt.

9. Mischungen nach Anspruch 7 oder 9, wobei der Gehalt an Isocyanatgruppen der Komponenten a1 und a2, bezogen auf die Gesamtmenge der Mischungskomponenten a1 und a2 0.1 bis 40 Gew.% beträgt.

10. Mischungen nach den Ansprüchen 7 bis 9, wobei das Verhältnis der Aktivierten Doppelbindungen zu den Isocyanatgruppen der Mischungskomponenten a1 und a2 50 : 1 bis 0,02 : 1 beträgt.

11. Zubereitungen, enthaltend
a) 1 bis 99 Gew.-% einer Verbindung gemäß Anspruch 1 oder einer Mischung gemäß Anspruch 7
b) 99 bis 1 Gew.-% einer von den Verbindungen gemäß Anspruch 1 verschiedenen Verbindung mit einer radikalisch polymerisierbaren C-C-Doppelbindung.

12. 2-Komponenten-Beschichtungsmittel nach den Ansprüchen 1 bis 11, enthaltend, bezogen auf die Verbindungen gemäß Anspruch 1 oder Mischungen gemäß Anspruch 7, Verbindungen mit mindestens einer gegenüber Isocyanatgruppen reaktiven Gruppe, mit der Maßgabe, daß das Verhältnis der Isocyanatgruppen zu den gegenüber Isocyanatgruppen reaktiven Gruppen 200 : 1 bis 0,5 : 1 beträgt.

13. Verfahren zur Herstellung von Verbindungen nach Anspruch 1, wobei man ein Diisocyanat mit einem Alkohol, der neben der Alkoholgruppe noch eine Aktivierte Doppelbindung trägt; bei einer Temperatur von 80 bis 280°C umsetzt.

14. Beschichtungsverfahren, **dadurch gekennzeichnet, daß** man einen Gegenstand mit einer Verbindung gemäß Anspruch 1, einer Mischung gemäß Anspruch 7, einer Zubereitung gemäß Anspruch 11 oder einem Beschichtungsmittel nach Anspruch 12 beschichtet, gegebenenfalls in einer Polyadditionsreaktion der Isocyanatgruppen bei Raumtemperatur oder unter erhöhter Temperatur härtet und gegebenenfalls mit energiereicher Strahlung zu Beginn, während oder nach der Polyadditionsreaktion bestrahlt.

15. Beschichtungsverfahren, **dadurch gekennzeichnet, daß** man einen Gegenstand mit einer Verbindung gemäß Anspruch 1, einer Mischung gemäß Anspruch 7, einer Zubereitung gemäß Anspruch 11 oder einem Beschichtungsmittel nach Anspruch 12 beschichtet und auf eine Temperatur bis 200°C erwärmt.

16. Beschichtungsverfahren, **dadurch gekennzeichnet, daß** man einen Gegenstand mit einer Verbindung gemäß Anspruch 1, einer Mischung gemäß Anspruch 7, einer Zubereitung gemäß Anspruch 11 oder einem Beschichtungsmittel nach Anspruch 12 beschichtet und gegebenenfalls nach Bestrahlung mit energiereicher Strahlung in einer Atmosphäre, die Wasserdampf, Ammoniak oder Amine enthält, aushärtet.

17. Verfahren zur Beschichtung von Gegenständen, wobei man
Ia. in Schritt Ia einen Gegenstand mit einem Film des Beschichtungsmittels gemäß Anspruch 12 beschichtet,
IIa. in Schritt IIa den Film des Beschichtungsmittels mit energiereicher Strahlung bestrahlt, wobei der Film vorgehärtet wird,
IIIa.in Schritt IIIa den Gegenstand, der mit dem vorgehärteten Film des Beschichtungsmittels beschichtet ist, mechanisch bearbeitet, insbesondere verformt oder die Oberfläche des vorgehärteten Films mit einem anderen Gegenstand in Kontakt bringt und
IVa.in Schritt IVa den vorgehärteten Film des Beschichtungsmittels, mit dem der bearbeitete Gegenstand beschichtet ist, endhärtet, indem man die Isocyanatgruppen in einer Polyadditionsreaktion reagieren läßt.

18. Verfahren zur Beschichtung von Gegenständen, wobei man
Ib. in Schritt Ib einen Gegenstand mit einem Film des Beschichtungsmittels gemäß Anspruch 12 beschichtet,
IIb.in Schritt IIb den Film des Beschichtungsmittels, mit dem der Gegenstand beschichtet ist, vorhärtet, indem man die Isocyanatgruppen in einer Polyadditionsreaktion reagieren läßt,
IIIb.in Schritt IIIb den Gegenstand, der mit dem vorgehärteten Film des Beschichtungsmittels beschichtet ist, mechanisch bearbeitet, insbesondere verformt oder die Oberfläche des vorgehärteten Films mit einem anderen Gegenstand in Kontakt bringt und
IVb.in Schritt IVb den vorgehärteten Film des Beschichtungsmittels mit energiereicher Strahlung bestrahlt, wobei der Film endgehärtet wird.

19. Beschichtete Gegenstände, erhältlich nach den Beschichtungsverfahren nach den Ansprüchen 14 bis 18.

20. Verfahren **dadurch gekennzeichnet, daß** man eine Verbindung gemäß Anspruch 1, eine Mischung gemäß Anspruch 7 oder eine Zubereitung gemäß Anspruch 11 als Gießharz, Spachtelmasse, Dichtungsmasse, Stereolithographieharz, Lötstopplack, strahlungshärtbare Massen für photopolymere Druckplatten und Fotoresiste, Druckfarbe, Klebstoff oder Dentalmasse oder als Harz für Verbundwerkstoffe einsetzt.

## Claims

1. A compound having isocyanate groups with or without blocking, allophanate groups and free-radically polymerizable C-C double bonds, the C-C double bonds being in activated form by virtue of a carbonyl group attached directly to them or by virtue of an oxygen atom in ether function (activated double bonds), derived from aliphatic or alicyclic polyisocyanates and alcohols A which in addition to the alcohol group also carry an activated double bond (compound I).

2. A compound as claimed in claim 1 of the formula I
OCN-R¹-(-R²-C(O)-R²-R¹-)ₙ-NCO I
in which
n is an integer from 1 to 10,
R¹ is a divalent aliphatic or alicyclic C₂-C₂₀ hydrocarbon unit
R² in each repeating unit is -NH- once and N-C(O)-R³ once, R³ being a radical derived from an alcohol A by abstracting the hydrogen atom of the alcoholic hydroxyl group A and the alcohol A carrying functional groups including, in addition to the alcohol group, an activated double bond.

3. A compound as claimed in claim 1 or 2, wherein the alcohol A is an ester of an aliphatic or aromatic polyol and acrylic or methacrylic acid, an amide of an amino alcohol and acrylic or methacrylic acid, or a vinyl ether derived from an aliphatic or aromatic polyol.

4. A compound as claimed in claim 3, wherein the aliphatic or aromatic polyol is a diol, triol or tetrol of 2 to 20 carbon atoms or a polyether polyol or polyester polyol or a polyacrylate polyol having an average OH functionality of from 2 to 10.

5. A compound as claimed in any of claims 1 to 4, wherein the radical R³ is derived from hydroxyethyl acrylate, hydroxypropyl acrylate, hydroxybutyl acrylate, hydroxyethyl methacrylate or 3-(acryloyloxy)-2-hydroxypropyl methacrylate.

6. A compound as claimed in any of claims 1 to 5, wherein the isocyanate is hexamethylene diisocyanate, isophorone diisocyanate, tetramethylxylylene diisocyanate, di(isocyanatocyclohexyl)methane or 1,3-bis(isocyanatomethyl)cyclohexane.

7. A mixture comprising
a1) from 1 to 100% by weight of compounds as claimed in claim 1
a2) from 0 to 99% by weight of another compound which in addition to one or more isocyanate groups includes a group selected from the series consisting of urethane, urea, biuret, allophanate, carbodiimide, uretonimine, uretdione and isocyanurate groups.

8. A mixture as claimed in claim 7, wherein the activated double bond content, based on the overall amount of the co-components a1 and a2, is from 0.02 to 20% by weight.

9. A mixture as claimed in claim 7 or 8, wherein the isocyanate group content of components a1 and a2, based on the overall amount of co-components a1 and a2, is from 0.1 to 40% by weight.

10. A mixture as claimed in any of claims 7 to 9, wherein the ratio of the activated double bonds to the isocyanate groups of the co-components a1 and a2 is from 50:1 to 0.02:1.

11. A formulation S comprising
a) from 1 to 99% by weight of a compound as claimed in claim 1 or of a mixture as claimed in claim 7, and
b) from 99 to 1% by weight of a compound which is different from the compounds as claimed in claim 1 and has a free-radically polymerizable C-C double bond.

12. A 2-component coating composition as claimed in any of claims 1 to 11, comprising, based on the compounds as claimed in claim 1 or mixtures as claimed in claim 7, compounds having at least one isocyanate-reactive group, with the proviso that the ratio of the isocyanate groups to the isocyanate-reactive groups is from 200:1 to 0.5:1.

13. A process for preparing a compound as claimed in claim 1, which comprises reacting a diisocyanate with an alcohol which in addition to the alcohol group carries an activated double bond at a temperature from 80 to 280°C.

14. A coating method which comprises coating an article with a compound as claimed in claim 1, a mixture as claimed in claim 7, a formulation as claimed in claim 11 or a coating composition as claimed in claim 12, if desired, curing the coating in a polyaddition reaction of the isocyanate groups at room temperature or at increased temperature, and, if desired, carrying out exposure to high-energy radiation at the beginning of, during or after the polyaddition reaction.

15. A coating method which comprises coating an article with a compound as claimed in claim 1, a mixture as claimed in claim 7, a formulation as claimed in claim 11 or a coating composition as claimed in claim 12 and heating it to a temperature of up to 200°C.

16. A coating method which comprises coating an article with a compound as claimed in claim 1, a mixture as claimed in claim 7, a formulation as claimed in claim 11 or a coating composition as claimed in claim 12 and, if desired, after carrying out exposure to high-energy radiation, curing it in an atmosphere which comprises water vapor, ammonia or amines.

17. A method of coating an article, which comprises
Ia. in step Ia, coating an article with a film of the coating composition as claimed in claim 12,
IIa.in step IIa, exposing the film of the coating composition to high-energy radiation, the film being precured,
IIIa.in step IIIa, mechanically working, especially deforming, the article coated with the precured film of the coating composition, or bringing the surface of the precured film into contact with another article, and
IVa. in step IVa, fully curing the precured film of the coating composition with which the worked article is coated, by reacting the isocyanate groups in a polyaddition reaction.

18. A method of coating an article, which comprises
Ib. in step Ib, coating an article with a film of the coating composition as claimed in claim 12,
IIb.in step IIb, procuring the film of the coating composition with which the article is coated by reacting the isocyanate groups in a polyaddition reaction,
IIIb.in step IIIb, mechanically working, especially deforming, the article coated with the precured film of the coating composition, or bringing the surface of the precured film into contact with another article, and
IVb.in step IVb, exposing the precured film of the coating composition to high-energy radiation, in the course of which the film is fully cured.

19. A coated article obtainable by a coating method as claimed in any of claims 14 to 18.

20. A process wherein a compound as claimed in claim 1, a mixture as claimed in claim 7 or a formulation as claimed in claim 11 is used as a casting resin, troweling compound, sealing compound, stereolithography resin, solder resist, radiation-curable compositions for photopolymeric printing plates and photoresists, printing ink, adhesive or dental compound or as a resin for composite materials.

## Revendications

1. Composés possédant des groupes isocyanate ou des groupes isocyanate coiffés, des groupes allophanate et des doubles liaisons C=C polymérisables par voie radicalaire, les doubles liaisons C=C étant activées par un groupe carbonyle auquel elles sont directement liées ou par un atome de O de la fonction éther (doubles liaisons activées), qui sont dérivés de polyisocyanates aliphatiques ou alicycliques et d'alcools A qui portent, outre le groupe alcool, une double liaison activée.

2. Composés selon la revendication 1 de formule I
OCN-R¹-(-R²-C(O)-R²-R¹-)ₙ-NCO I
dans laquelle
n représente un nombre entier allant de 1 à 10,
R¹ représente un motif hydrocarbure en C₂ à C₂₀ aliphatique ou alicyclique divalent,
R² représente, dans chaque motif récurrent, une fois -NH- et une fois N-C(O)-R³, R³ représentant un groupe dérivé d'un alcool A par abstraction de l'atome de H à partir du groupe hydroxyle alcoolique, où l'alcool A porte, en tant que groupe fonctionnel, outre le groupe alcool, une double liaison activée.

3. Composés selon la revendication 1 ou 2, dans lesquels l'alcool A est un ester d'un polyol aliphatique ou aromatique et d'un acide acrylique ou méthacrylique, un amide d'un aminoalcool et d'un acide acrylique ou méthacrylique, ou un éther vinylique dérivé d'un polyol aliphatique ou aromatique.

4. Composés selon la revendication 3, dans lesquels le polyol aliphatique ou aromatique est un diol, un triol ou un tétrol possédant 2 à 20 atomes de C, ou bien un polyéther-polyol ou un polyester-polyol, ou encore un polyacrylate-polyol présentant une fonctionnalité OH moyenne allant de 2 à 10.

5. Composés selon les revendications 1 à 4, dans lesquels le groupe R³ est dérivé de l'acrylate d'hydroxyéthyle, de l'acrylate d'hydroxypropyle, de l'acrylate d'hydroxybutyle, du méthacrylate d'hydroxyéthyle ou du méthacrylate de 3-(acryloyloxy)-2-hydroxypropyle.

6. Composés selon les revendications 1 à 5, dans lesquels l'isocyanate est le diisocyanate d'hexaméthylène, le diisocyanate d'isophorone, le diisocyanate de tétraméthylxylylène, le di(isocyanatocyclohexyl)-méthane ou le 1,3-bis-(isocyanatométhyl)-cyclohexane.

7. Mélanges contenant
a1) 1% à 100% en poids de composés selon la revendication 1,
a2) 0% à 99% en poids d'un autre composé qui possède, outre un ou plusieurs groupes isocyanate, un autre groupe choisi dans le groupe formé par les groupes uréthanne, urée, biuret, allophanate, carbodiimide, urétonimine, uretdione et isocyanurate.

8. Mélanges selon la revendication 7, dans lesquels la teneur en double liaisons activées va de 0,02% à 20% en poids par rapport à la quantité totale des composants a1 et a2 du mélange.

9. Mélanges selon la revendication 7 ou 8, dans lesquels la teneur en groupes isocyanate des composants a1 et a2 va de 0,1% à 40% en poids par rapport à la quantité totale des composants a1 et a2 du mélange.

10. Mélanges selon les revendications 7 à 9, dans lesquels le rapport des doubles liaisons activées aux groupes isocyanate des composants a1 et a2 du mélange va de 50:1 à 0,02:1.

11. Préparations contenant
a) 1% à 99% en poids d'un composé selon la revendication 1 ou d'un mélange selon la revendication 7,
b) 1% à 99% en poids d'un composé différent des composés selon la revendication 1 qui possède une double liaison C=C polymérisable par voie radicalaire.

12. Matériaux de revêtement à deux composants selon les revendications 1 à 11, contenant, par rapport aux composés selon la revendication 1 ou aux mélanges selon la revendication 7, des composés possédant au moins un groupe réactif envers les groupes isocyanate, avec la condition que le rapport des groupes isocyanate aux groupes réactifs envers les groupes isocyanate aille de 200:1 à 0,5:1.

13. Procédé pour la préparation de composés selon la revendication 1, dans lequel on fait réagir, à une température allant de 80°C à 280°C, un diisocyanate avec un alcool qui porte, outre le groupe alcool, une double liaison activée.

14. Procédé de revêtement, **caractérisé en ce que** l'on revêt un objet avec un composé selon la revendication 1, un mélange selon la revendication 7, une préparation selon la revendication 11 ou un matériau de revêtement selon la revendication 12, **en ce que** l'on réalise éventuellement un durcissement au moyen d'une réaction de polyaddition des groupes isocyanate à température ambiante ou à une température élevée, et **en ce que** l'on irradie éventuellement avec un rayonnement riche en énergie, au début, pendant ou après la réaction de polyaddition.

15. Procédé de revêtement, **caractérisé en ce que** l'on revêt un objet avec un composé selon la revendication 1, un mélange selon la revendication 7, une préparation selon la revendication 11 ou un matériau de revêtement selon la revendication 12, et **en ce que** l'on chauffe à une température allant jusqu'à 200°C.

16. Procédé de revêtement, **caractérisé en ce que** l'on revêt un objet avec un composé selon la revendication 1, un mélange selon la revendication 7, une préparation selon la revendication 11 ou un matériau de revêtement selon la revendication 12, et **en ce que** l'on procède à un durcissement, éventuellement après une irradiation avec un rayonnement riche en énergie, sous une atmosphère contenant de la vapeur d'eau, de l'ammoniac ou des amines.

17. Procédé pour le revêtement d'objets, dans lequel
Ia. on revêt, dans une étape Ia, un objet avec un film du matériau de revêtement selon la revendication 12,
IIa. on irradie, dans une étape IIa, le film du matériau de revêtement avec un rayonnement riche en énergie, le film étant ainsi prédurci,
IIIa. on soumet à un traitement mécanique, en particulier à une déformation, dans une étape IIIa, l'objet que l'on a revêtu avec le film prédurci du matériau de revêtement, ou bien on met en contact la surface du film prédurci avec un autre objet, et
IVa. on finit de durcir, dans une étape IVa, le film prédurci du matériau de revêtement avec lequel on a revêtu l'objet traité en faisant réagir les groupes isocyanate dans une réaction de polyaddition.

18. Procédé pour le revêtement d'objets, dans lequel
Ib. on revêt, dans une étape Ib, un objet avec un film d'un matériau de revêtement selon la revendication 12,
IIb. on prédurcit, dans une étape IIb, le film du matériau de revêtement avec lequel on a revêtu l'objet en faisant réagir les groupes isocyanate dans une réaction de polyaddition,
IIIb. on soumet à un traitement mécanique, en particulier à une déformation, dans une étape IIIb, l'objet que l'on a revêtu avec le film prédurci du matériau de revêtement, ou bien on met en contact la surface du film prédurci avec un autre objet, et
IVb. on irradie, dans une étape IVb, le film prédurci du matériau de revêtement avec un rayonnement riche en énergie, pour obtenir ainsi son durcissement final.

19. Objets revêtus que l'on peut obtenir au moyen des procédés de revêtement selon les revendications 14 à 18.

20. Procédé, **caractérisé en ce que** l'on met en oeuvre un composé selon la revendication 1, un mélange selon la revendication 7 ou une préparation selon la revendication 11, en tant que résine de coulée, mastic, matériau d'étanchéité, résine pour stéréolithographie, réserve de soudure, matériaux radiodurcissables pour des plaques d'impression photopolymères et des photorésists, encre d'impression, colle ou matériau dentaire ou en tant que résine pour des matériaux composites.
